# FASCICULE DE BREVET EUROPEEN

(11) **EP 1 968 993 B1**
(45) Date de publication et mention de la délivrance du brevet: **11.01.2012**
(21) Numéro de dépôt: 06841375.6
(22) Date de dépôt: 14.12.2006
(51) Int. Cl.: C07H 15/203, C07H 15/207, C07D 339/04, C07C 403/14, C07C 403/20, A61K 8/67, A61Q 19/02, C07D 311/72

(54) **NOUVEAUX COMPOSÉS POLYINSATURÉS, LEUR PROCÉDÉ DE PRÉPARATION ET LES COMPOSITIONS LES CONTENANT**
NEUE MEHRFACH UNGESÄTTIGTE VERBINDUNGEN, VERFAHREN ZU DEREN HERSTELLUNG ZUSAMMENSETZUNGEN, DIE DIESE ENTHALTEN
NOVEL POLYUNSATURATED COMPOUNDS, METHOD FOR PREPARING SAME AND COMPOSITIONS CONTAINING SAME

(30) Priorité: 14.12.2005 FR 0512661
(43) Date de publication de la demande: 17.09.2008
(73) Titulaire: Pierre Fabre Dermo-Cosmétique, 92100 Boulogne-Billancourt (FR)
(72) Inventeur: BORDAT, Pascal, F-31320 Mervilla (FR); TARROUX, Roger, F-31300 Toulouse (FR); SAURAT, Jean-Hilaire, CH-1206 Geneve (CH); SORG, Olivier, CH-1205 Geneve (CH); BRAYER, Jean-Louis, F-60440 Nanteuil Le Haudoin (FR); FRISON, Natacha, F-60100 Creil (FR)
(74) Mandataire: Ahner, Francis
(86) Numéro de dépôt international: PCT/EP2006/069731
(87) Numéro de publication internationale: WO 2007/068745

(56) Documents cités:
- WO-A-02/03912
- FR-A- 2 791 679
- US-A- 5 962 534

## Description

La présente invention concerne de nouveaux composés dérivés de rétinoïdes, leurs procédés de préparation, les compositions les contenant, et leurs utilisations dans le domaine cosmétique et/ou dermatologique.

Les dérivés rétinoïques sont aujourd'hui utilisés en dermatologie dans différentes indications comme le psoriasis ou l'ichtyose ou bien pour obtenir une dépigmentation de la peau (réduction de la mélanogénèse sous l'action de la vitamine A).

Cependant l'utilisation des dérivés rétinoïques par voie topique se heurte à un certain nombre de difficultés du fait du manque de stabilité dans le temps et à la lumière de ces dérivés, de l'irritation résultant de sur-concentrations locales ainsi que d'une faible pénétration de ces dérivés au travers de la couche cornée. Ce dernier inconvénient est dû à la grande lipophilie de la substance qui déposée sur la peau est en fait en grande partie éliminée avec la desquamation.

Les effets secondaires, rougeurs, irritation, oedèmes et desquamation excessive, limitent l'utilisation de ces dérivés rétinoïques à des patients réellement motivés. D'où l'intérêt d'améliorer la biodisponibilité de l'actif et sa pénétration en évitant les effets néfastes des sur-concentrations locales.

La demanderesse a proposé par le passé de s'affranchir des effets secondaires de ces composés en utilisant des complexes glycosylés ternaires aptes à libérer la substance active sous l'action de deux enzymes (FR 2 791 679). On obtient ainsi un relargage lent qui évite les effet d'accumulation.

US 5962534 décrit l'utilisation du 4"-hydroxy rétinol pour la réduction de la pigmentation de la peau

Il subsiste toutefois le besoin de trouver des composés possédant des effets secondaires moins gênants, ou alternativement dont l'activité renforcée permettrait une utilisation dans des proportions plus faibles pour lesquelles les effets indésirables deviendront moins gênants.

La Demanderesse a ainsi mis en évidence que des nouveaux dérivés de l'acide rétinoïque possèdent une remarquable activité dépigmentante.

La présente invention concerne les composés de formule (I) : 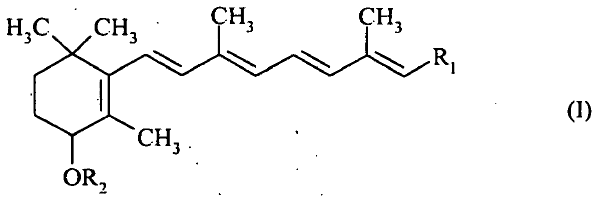 dans laquelle
R₁ représente un groupement R'₁ ou -A-R'₁, dans lesquels R'₁ est choisi parmi - COOH, -COOR₃, -CONH₂, -CONHR₃, -CONR₃R₄, -CHO, -CH₂OH, -CH₂OR₅, et A représente un groupement alkylène C₁-C₁₆ linéaire ou ramifié, alkénylène C₂-C₁₆ linéaire ou ramifié, ou alkynylène C₂-C₁₆ linéaire ou ramifié,
R₂ représente :
- un groupement aryle éventuellement substitué ou hétéroaryle éventuellement substitué ou,
- un résidu osidique ou,
- un résidu d'acide gras éventuellement ramifié et/ou substitué de préférence en bout de chaîne et en particulier par un radical hydroxy, acétoxy ou par un radical amino protégé ou non,
- un groupe -OC -(CH₂)ₙ - CO -tocophéryle (alpha, bêta ou gamma ou delta), avec 2≤ n ≤ 10,
- un groupe -R'₂-O-R₆, dans lequel R'₂ est un groupement arylène éventuellement substitué, et R₆ représente un atome d'hydrogène, un groupement alkyle C₁-C₆ linéaire ou ramifié , ou un résidu osidique,
R₃ et R₄ représentent indépendamment un radical alkyle C₁-C₁₆ linéaire ou ramifié éventuellement substitué, alkényle C₂-C₁₆ linéaire ou ramifié, ou alkynyle C₂-C₁₆ linéaire ou ramifié éventuellement substitué,
R₅ représente un radical alkyle C₁-C₁₆ linéaire ou ramifié éventuellement substitué, alkynyle C₂-C₁₆ linéaire ou ramifié éventuellement substitué, alkynyle C₂-C₁₆ linéaire ou ramifié éventuellement substitué, ou acyle C₂-C₁₆ linéaire ou ramifié éventuellement substitué,
leurs énantiomères, diastéréoisomères, ainsi que leurs éventuels sels d'addition à un acide ou à une base physiologiquement acceptable.

Parmi les acides pharmaceutiquement acceptables, on peut mentionner de façon non limitative, les acides chlorhydrique, hydrobromique, sulfurique, phosphonique, acétique, trifluoroacétique, lactique, pyruvique, malonique, succinique, glutarique, fumarique, tartrique, maléique, méthanesulfonique, camphorique, oxalique, ...

Parmi les bases pharmaceutiquement acceptables, on peut mentionner de façon non limitative, l'hydroxyde de sodium, l'hydroxyde de potassium, la triéthylamine, la tert-butylamine, ...

Les doubles liaisons du motif insaturé sont en général toutes de configuration E, mais la présente invention concerne aussi l'utilisation de composés de formule (1) pour lesquels toutes ou une partie seulement des doubles liaisons présentent une configuration Z.

Le terme aryle désigne un groupement phényle, naphtyle, ou biphényle.

Le terme hétéroaryle désigne un groupement mono- ou bicyclique, contenant au moins un cycle aromatique, de 5 à 11 chaînons, contenant de 1 à 5 hétéroatomes choisis parmi azote, oxygène et soufre. On peut citer à titre d'exemple et de façon non limitative les groupements pyridyle, pyridazinyle, pyrazinyle, pyrimidinyle, thiényle, furyle, pyrrolyle, imidazolyle, pyrazolinyle, indolyle, benzimidazolyle, oxazolyle, thiazolyle, isothiazolyle, ...

La terminaison « -ène » signifie que le groupement concerné est un radical bivalent possédant la même définition que le radical de base, tant dans l'aspect le plus général de la présente invention, que dans les aspects particuliers, avantageux et/ou préférés.

L'expression « éventuellement substitué » associée aux groupements aryle, hétéroaryle, et tocophéryle, signifie que ces groupements sont non substitués ou substitués par un ou plusieurs atomes d'halogène, ou groupements alkyle (C₁-C₆) linéaire ou ramifié, alkényle (C₂-C₆), linéaire ou ramifié, alkynyle (C₂-C₆) linéaire ou ramifié, alkoxy (C₁-C₁₆) linéaire ou ramifié, hydroxy, trialkylsilyle (C₃-C₁₈) linéaire ou ramifié, mercapto, alkylthio, cyano, amino (éventuellement substitué par un ou deux groupements alkyle (C₁-C₆) linéaire ou ramifié), nitro, carboxy, formyle, alkoxycarbonyle, aminocarbonyle (éventuellement substitué par un ou deux groupements alkyle (C₁-C₆) linéaire ou ramifié), et carbamoyle, étant entendu que les groupements hétéroaryles peuvent en plus être substitués par un groupement oxo.

L'expression « éventuellement substitué » associée aux groupements alkyle, alkényle, alkynyle, signifie que ces groupements sont non substitués ou substitués par un ou plusieurs atomes d'halogène ou groupement alkoxy (C₁-C₁₆) linéaire ou ramifié, hydroxy, mercapto, alkylthio, cyano, amino (éventuellement substitué par un ou deux groupements alkyle (C₁-C₆) linéaire ou ramifié), nitro, carboxy, formyle, alkoxycarbonyle, aminocarbonyle (éventuellement substitué par un ou deux groupements alkyle (C₁-C₆) linéaire ou ramifié), et carbamoyle.

Le terme résidu d'acide aminé désigne des résidus d'acides α-aminés naturels tels que Ala, Arg, Asn, Asp, Cys, Gln, Glu, Gly, His, Ile, Leu, Lys, Met, Phe, Pro, Ser, Thr, Trp, Tyr, et Val, ou des résidus d'acides aminés non naturels tels que la β-alanine, l'allylglycine, ...

Le terme résidu osidique désigne un radical correspondant à un dérivé de sucre (ou d'hydrate de carbone) tels que glucose, galactose, fructose, mannose, fucose, rhamnose, étant entendu que dans ces résidus, un ou plusieurs groupements hydroxy peuvent être protégés par un groupement classiquement utilisé en ce sens comme par exemple les groupements benzyle, acétyle, benzoyle.

Le terme résidu d'acide gras désigne un radical correspondant à un acide gras, linéaire ou ramifié comportant de 4 à 30 atomes de carbone. Ce radical peut provenir d'un acide gras essentiel, ou d'un acide gras non essentiel. On peut citer à titre d'exemple les résidus d'acide butyrique, valérianique, capronique, caprylique, caprique, laurique, myristique, palmitique, stéarique, arachinique, béhénique, oléïque, linoléïque, linolénique, arachidonique, azélaïque, lipoïque.

Ces radicaux peuvent être non substitués ou substitués par un ou plusieurs atomes d'halogène ou groupement alkoxy (C₁-C₁₆) linéaire ou ramifié, hydroxy éventuellement estérifié (préférentiellement acetylé), mercapto, alkylthio, cyano, amino protégé ou non (éventuellement substitué par un ou deux groupements alkyle (C₁-C₆) linéaire ou ramifié), nitro, carboxy, formule, alkoxycarbonyle, aminocarbonyle (éventuellement substitué par un ou deux groupements alkyle (C₁-C₆) linéaire ou ramifié), et carbamoyle.

Le radical tocophéryle peut dériver de toutes les formes de tocophérol : alpha, bêta, gamma ou delta

Compte tenu du caractère chiral de ces résidus, la configuration du carbone anomérique peut-être α, β, ou un mélange α-β en proportions variables.

Dans le cadre de la présente invention, le groupement aryle préféré est le groupement phényle.

Les radicaux alkyles présents dans les composés de formule (I) selon l'invention sont préférentiellement des radicaux alkyles (C₁-C₆) linéaires ou ramifiés. On peut citer en particulier les groupements méthyle, éthyle, propyle, isopropyle, butyle, *sec*-butyle, *tert*-butyle, pentyle, isopentyle, hexyle.

Les radicaux alkényles présents sur les composés de formule (I) selon l'invention seront avantageusement des radicaux alkényles (C₂-C₆) linéaires ou ramifiés, par exemple on peut citer les groupements allyle, ou vinyle.

Les radicaux alkynyles présents sur les composés de formule (I) selon l'invention seront avantageusement des radicaux alkynyles (C₂-C₆) linéaires ou ramifiés, par exemple alcyne, propyne, butyne.

Dans les composés de formule (I), le groupement R₁ représente avantageusement un groupement R'₁ choisi parmi -COOH, -COOR₃, -CHO, -CH₂OH, -CH₂OR₅, dans lesquels R₃ et R₅ sont tels que définis précédemment.

Conformément à une autre caractéristique avantageuse de l'invention, dans le composé de formule générale (I) A représente un groupement méthylène.

Des composés convenant bien à la mise en oeuvre de la présente invention sont les composés de formule (I) pour lesquels R₂ représente un groupement -R'₂-O-R₆ dans lequel R'₂ est un groupement arylène éventuellement substitué, et R₆ représente un groupement alkyle (C₁-C₆) linéaire ou ramifié, ou un résidu osidique.

D'autres composés de formule (I) convenant bien à la mise en oeuvre de la présente invention sont les composés pour lesquels R₂ représente un groupement -R'₂-O-R₆ dans lequel R'₂ est un groupement phénylène et R₆ représente un résidu osidique, préférentiellement un résidu glucose, galactose, fructose, mannose, fucose, rhamnose.

Parmi les composés de formule (1) on préfèrera plus particulièrement les composés suivants :
- l'acide (2E,4E,6E,8E)-3,7-diméthyl-9-{2,6,6-triméthyl-3-[4-((2S,3R,4S, 5S,6R)-3,4,5-trihydroxy-6-hydroxyméthyl-tetrahydro-pyran-2-yloxy)-phénoxy]-cyclohex-1-ényl}-nona-2,4,6,8-tétraènoique, 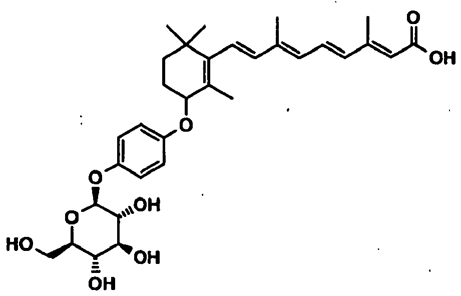
- l'ester tert-butylique de l'acide (2E,4E,6E,8E)-9-[3-(4-méthoxy-phénoxy)-2,6,6-triméthyl-cyclohex-1-ényl]-3,7-diméthyl-nona-2,4,6,8-tétraènoique, 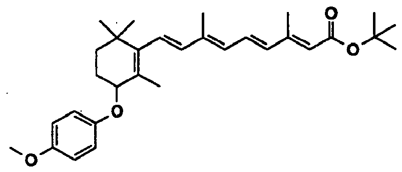
- le (2E,4E,6E,8E)-9-[3-(4-méthoxy-phénoxy)-2,6,6-triméthyl-cyclohex-1-ényl]-3,7-diméthyl-nona-2,4,6,8-tétraènal, 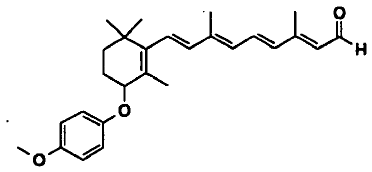
- l'ester tert-butylique de l'acide (2E,4E,6E,8E)-9-[3-(4-hydroxy-phénoxy)-2,6,6-triméthyl-cyclohex-1-ényl]-3,7-diméthyl-nona-2,4,6,8-tétraènoique. 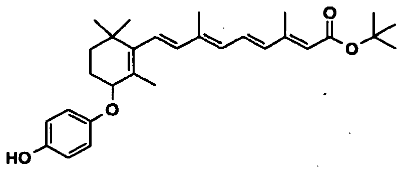
- le (2E, 4E, 6E, 8E)-9-[3-(4-hydroxy-phenoxy)-2,6,6-trimethyl-cyclohex-1-ényl]-3,7-dimethyl-nona-2,4,6,8-tetraènal 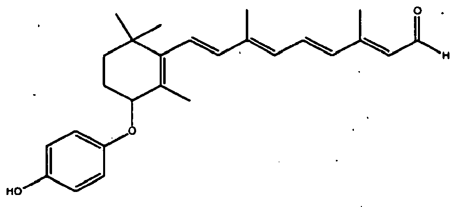
   - le (2E, 4E, 6E, 8E)-3,7-diméthyl-9-{2,6,6 triméthyl-3-diméthyl-[4-((2S, 3R, 4S, 5S, 6R)-3,4,5-trihydroxy-6-hydroxyméthyl-tétrahydro-pyran-2-yloxy)-phénoxy]- cyclohex-1-enyl}-nona-2,4,6,8-tetraènal, 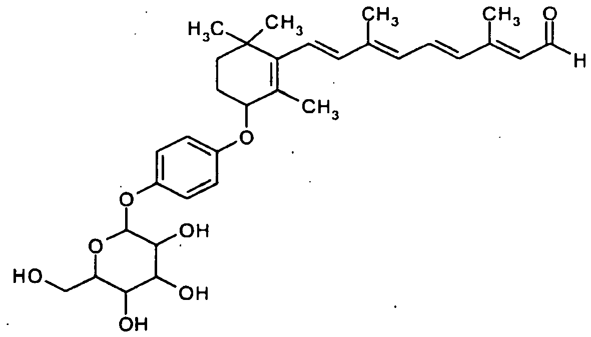
   - le 9-[(3-trans décènoate)-2,6,6 triméthyl-cyclohex-1-ényl]-3,7-diméthylnona-2,4,6,8 tétraènal, 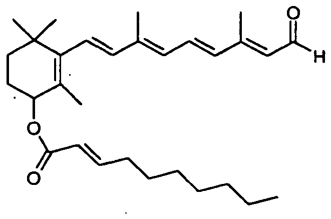
   - le 9-[(3-oléate)-2,6,6-triméthyl-cyclohex-1-ényl]-3,7-diméthyl-nona-2,4,6,8 tétraènal, 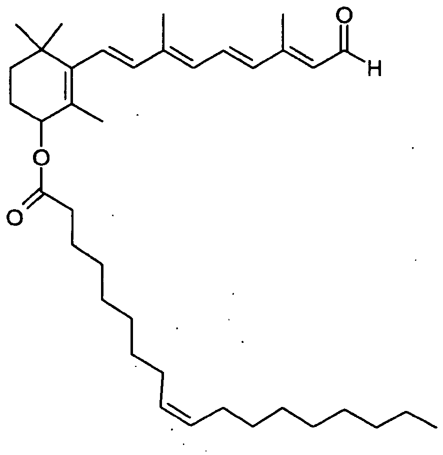
   - le 9-[(3-linoléate)-2,6,6-triméthyl-cyclohex-1-ényl]-3,7-diméthyl-nona-2,4,6,8-tétraènal, 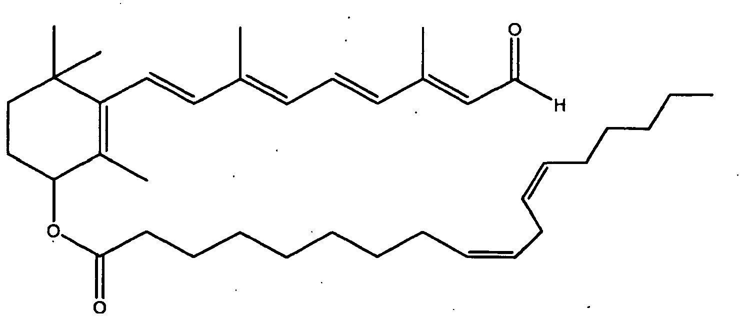
   - le 9-[(3-linolénate)-2,6,6-triméthyl-cyclohex-1-ényl]-3,7-diméthylnona-2,4,6,8-tétraènal, 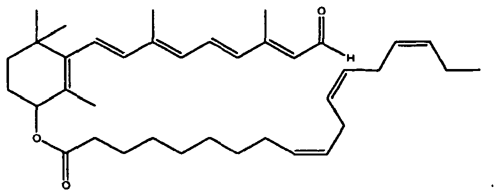
   - le 9-[(3-lipoate)-2,6,6-triméthyl-cyclohex-1-ényl]-3,7-diméthylnona-2,4,6,8-tétraènal, 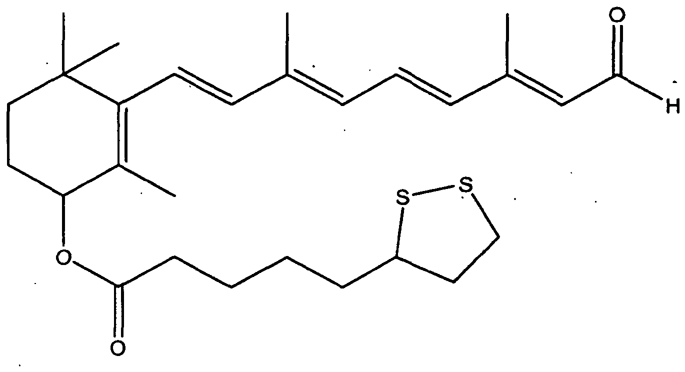
   - le 9-[{3-(8-hydroxy-5-méthyl-2-octénoate)}-2,6,6-triméthyl-cyclohex-1-ényl]-3,7-diméthyl-nona-2,4,6,8-tétraènal 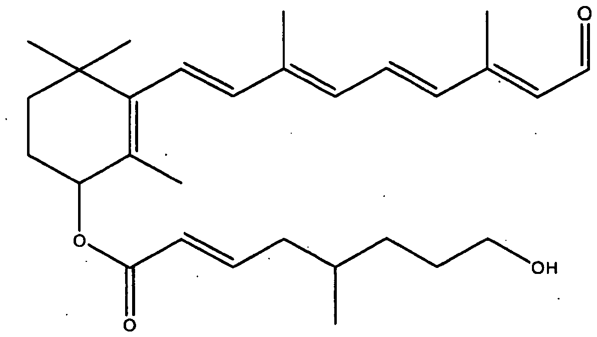
   - l'ester 3-((1E,3E,5E,7E)-3,7-dimethyl-9-oxo-nona-1,3,5,7-tetraenyl)-2,4,4-trimethyl-cyclohex-2-enyl de l'acide (E)-14-Hydroxy-tetradec-2-enoique 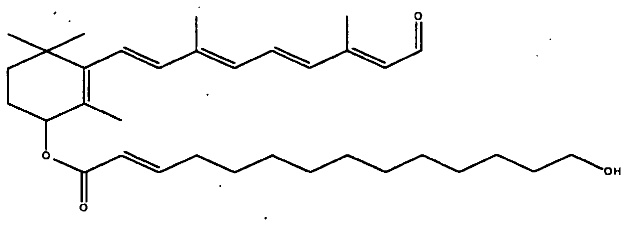
   - l'ester 3-((1E,3E,5E,7E)-3,7-dimethyl-9-oxo-nona-1,3,5,7-tetraenyl)-2,4,4-trimethyl-cyclohex-2-enyl de l'acide (E)-10-Hydroxy-dec-2-enoique 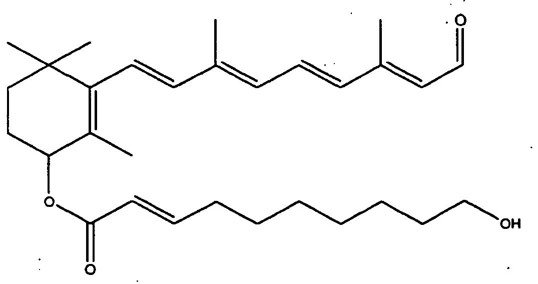
   - l'ester 3-((1E,3E,5E,7E)-3,7-dimethyl-9-oxo-nona-1,3,5,7-tetraenyl)-2,4,4-trimethyl-cyclohex-2-enyl de l'acide (E)-10-Acetoxy-dec-2-enoique 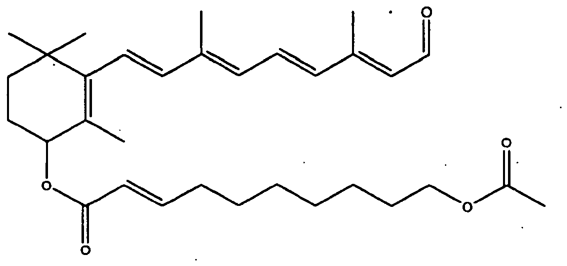
   - le 9-[(3-tétraacétyl glucose)-2,6,6-triméthyl-cyclohex-1-ényl]-3,7-diméthylnona-2,4,6,8-tétraènal 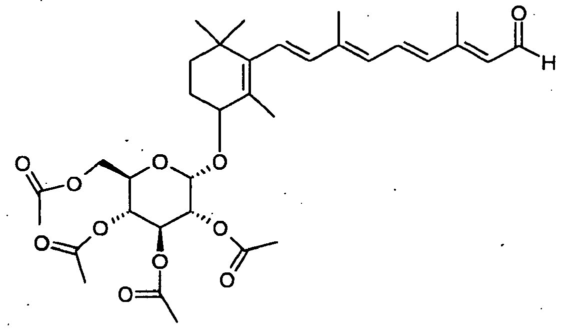
   - Bis ester 3-((1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R, 8R)-4,8,12-trimethyltridecyl)-1-benzopyran-6-yl de l'acide heptane dioique 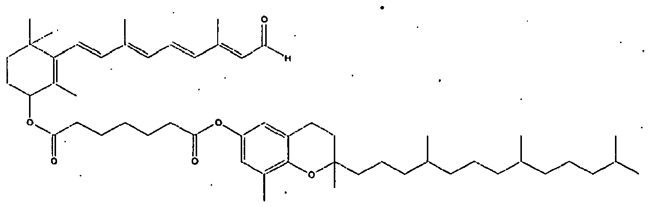
   - Bis ester 3-((1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R,8R)-4,8, 12-trimethyltridecyl)-1-benzopyran-6-yl de l'acide nonadioique 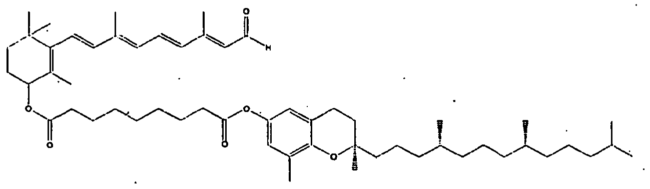
   - Bis ester 3-((1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R,8R)-4,8,12-trimethyltridecyl)-1-benzopyran-6-yl de l'acide succinique 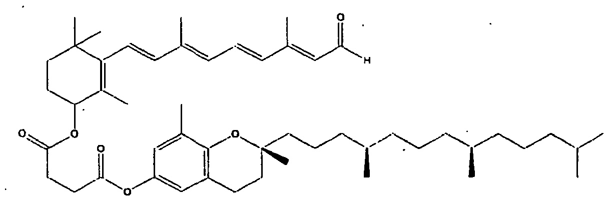
   - Bis ester 3-((1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R,8R)-4,8,12-trimethyltridecyl)-1-benzopyran-6-yl de l'acide pentanedioique

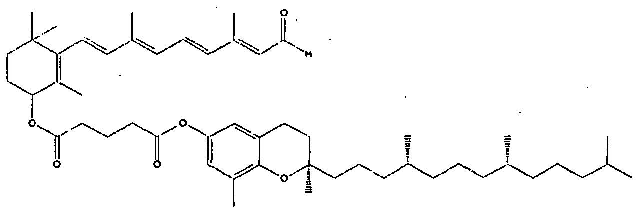

La présente invention concerne également le procédé de préparation des composés de formule (I), caractérisé en ce que le composé de formule (II) : 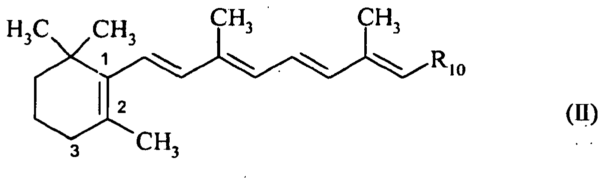 dans laquelle R₁₀ a la même signification que le radical R₁ défini précédemment, à l'exception du groupement -CH₂OH, est soumis à une réaction d'oxydation allylique en position 3, pour conduire à un composé de formule (III) : 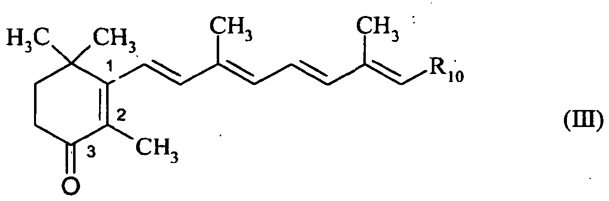 dans laquelle R₁₀ est tel que défini précédemment,
dont la fonction carbonyle en position 3 est ensuite réduite en alcool correspondant de formule (IV) : 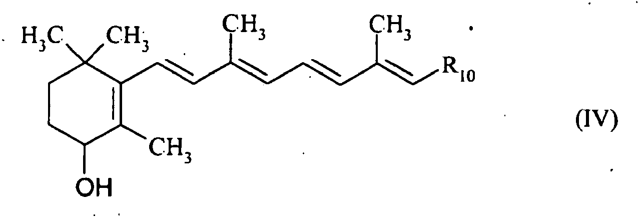 composé (IV) qui subit une réaction d'alkylation ou de couplage en milieu alcalin, acide ou neutre, éventuellement en présence d'un agent de couplage, à l'aide d'un réactif de formule R₂-X dans laquelle X représente un groupement hydroxy, ou un atome d'halogène, étant entendu que le groupement hydroxy peut être si nécessaire activé sous la forme d'un groupe partant,
pour conduire à un composé de formule (Ia) : 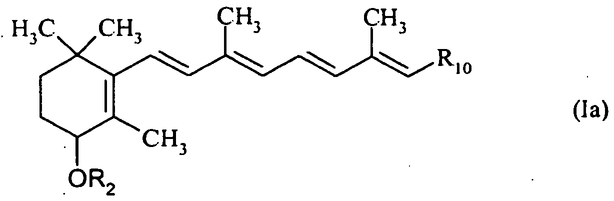 cas particulier des composés de formule (I) pour lequel R₂ est tel que défini précédemment, et R₁₀ a la même signification que ci-dessus,
qui, lorsque le radical R₁₀ représente un groupement -COOR₃, peut subir une réaction d'hydrolyse ou de réduction pour conduire au composé de formule (1b) : 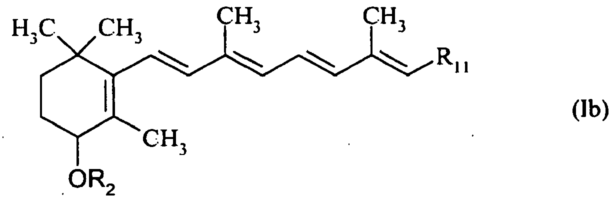 cas particulier des composés de formule (I) pour lequel R₂ est tel que défini précédemment, et R₁₁ représente un groupement -COOH, ou CH₂OH,
étant entendu que les différents groupements présents sur les composés précédents peuvent être à tout moment opportun de la synthèse protégés puis déprotégés en fonction de leurs incompatibilité avec les réactifs utilisés. Les groupements protecteurs susceptibles d'être utilisés dans le cadre de la présente invention sont choisis parmi les groupements classiquement utilisés, tels que ceux décrits par exemple dans « Protective Group in Organic Synthesis, Wiley-Interscience, 3rd Edition, 1999».

La réaction d'oxydation en position 3 du composé de formule (II) est réalisée à l'aide des oxydants classiques pour les positions allyliques, parmi lesquels on peut citer l'oxyde de sélénium, l'hypochlorite de sodium, l'oxyde de ruthénium, l'oxyde de chrome, et l'oxyde de manganèse. De préférence on utilisera l'oxyde de manganèse. La réaction peut se conduire dans des solvants variés tels que le dichlorométhane, le dichloro-1,2-éthane, le tétrahydrofurane, le pentane, ou l'hexane. La température du milieu réactionnel peut être basse, ambiante, ou à reflux, en fonction du solvant choisi.

La réduction de la fonction carbonyle du composé de formule (III) est réalisée préférentiellement à l'aide d'hydrures, tels que les hydrures d'aluminium, de bore, et plus particulièrement les hydrures d'aluminium encombrés et désactivés, à basse température. De façon avantageuse, on choisira l'hydrure de diisobutylaluminium (DIBAL-H), dans des conditions de températures variant ente -20°C et -70°C, dans un solvant éthéré tel que le tétrahydrofurane ou le dioxanne, mais aussi dans le toluène.

La réaction d'alkylation du composé de formule (IV) pour conduire aux composés de formule (Ia) ou (Ib) sera par exemple une réaction de Mitsunobu dans laquelle l'agent d'alkylation est un phénol activé par une combinaison de phosphine et de diazocarboxylate, et plus spécifiquement de triphénylphosphine, et de diazodicarboxylate de diisopropyle (DIAD).

L'hydrolyse ou la réduction optionnelle du groupement R₁₀ présent dans les composés de formule (la) peut se faire à l'aide de réactifs classiques, comme par exemple le fluorure de tétrabutylammonium pour l'hydrolyse, et le DIBAL-H pour la réduction.

L'invention concerne également l'utilisation de composés de formule (IIIa) : 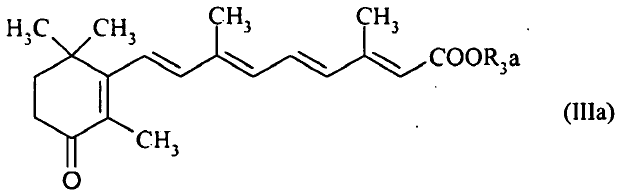 dans laquelle le groupement R₃a représente un groupement alkyle C₃-C₁₆ ramifié comme intermédiaire de synthèse pour l'obtention de certains composés de formule (I).

L'invention concerne aussi l'utilisation de composés de formule (IVa) : 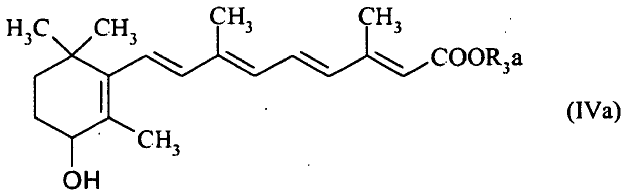 dans laquelle R₃a représente un groupement alkyle C₃-C₁₆ ramifié en tant qu'intermédiaire de synthèse pour l'obtention de certains composé de formule (I).

Dans un aspect avantageux, dans les composés de formule (IIIa) et (IVa) le groupement R₃a représente un groupement tert-butyle.

Parmi ces composés (IIIa, IVa), on peut mentionner plus particulièrement :
- l'ester tert-butylique de l'acide (2E,4E,6E,8E)-3,7-diméthyl-9-(2,6;6-triméthyl-3-oxo-cyclohex-1-ényl)-nona-2,4,6,8-tétraènoique, 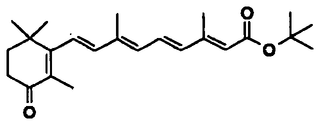
- l'ester tert-butylique de l'acide (2E,4E,6E,8E)-9-(3-Hydroxy-2,6,6-triméthyl-cyclohex-1-ényl)-3,7-diméthyl-nona-2,4,6,8-tétraènoique.

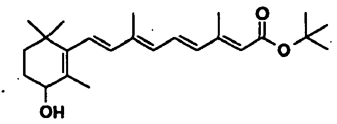

Les composés de formule (I) telle que définie ci-dessus ont montré de bonnes capacités à inhiber la production de la mélanine par les mélanocytes, démontrant ainsi leur intérêt dans les domaines cosmétique et/ou dermatologique.

La présente invention concerne ainsi une composition, caractérisée en ce qu'elle comprend au moins un composé de formule (I) telle que définie ci-dessus, dans un milieu physiologiquement acceptable.

La composition selon l'invention est notamment destinée à une application topique; elle comprend par ailleurs un milieu physiologiquement acceptable, c'est-à-dire compatible avec la peau y compris éventuellement le cuir chevelu, les muqueuses, les cheveux, les poils et/ou les yeux et peut constituer notamment une composition cosmétique ou dermatologique.

Les dérivés selon l'invention, seuls ou en mélange, ainsi que la composition les comprenant, peuvent être utilisés en application topique sur la peau, les poils et/ou les cheveux.

La quantité de dérivés utilisable dans le cadre de l'invention dépend bien évidemment de l'effet recherché.

A titre d'exemple, cette quantité peut aller par exemple de 0,01% à 5% en poids, de préférence de 0,05 % à 0,5 % en poids, par rapport au poids total de la composition.

Les composés de formule (1) ont l'avantage d'être très peu toxiques, et ont montré de bonnes propriétés inhibitrices de la production de mélanine par les mélanocytes. De plus ils présentent des effets secondaires moindres (sécheresse de la peau et inflammation) que des produits de référence utilisés couramment comme par exemple l'acide rétinoïque ou d'autres acides de synthèse du même type. En outre leur stabilité se trouve également améliorée.

Un autre aspect de l'invention concerne un procédé cosmétique de blanchiment et/ou d'éclaircissement de la peau humaine et/ou des poils et/ou des cheveux comprenant l'application sur la peau et/ou les poils et/ou les cheveux, d'une composition cosmétique contenant au moins un composé de formule (I) telle que définie précédemment.

L'invention concerne également un procédé cosmétique pour éliminer les taches pigmentaires brunâtres et/ou les taches de sénescence de la peau humaine, comprenant l'application sur la peau, d'une composition cosmétique contenant au moins un composé de formule (I) telle que définie précédemment.

Dans une variante, l'invention concerne l'utilisation d'au moins un composé de formule (I) en tant que médicament.

Plus particulièrement, l'invention concerne l'utilisation d'au moins un composé de formule (I) telle que définie précédemment, pour la fabrication d'une composition dermatologique destinée à dépigmenter la peau et/ou les poils et/ou les cheveux.

L'invention concerne aussi les compositions pharmaceutiques comprenant au moins un composé de formule (1) telle que définie précédemment, seul ou en combinaison avec un ou plusieurs excipients pharmaceutiquement acceptables.

Parmi les compositions pharmaceutiques selon l'invention, on peut mentionner plus particulièrement celles convenant à une administration orale, parentérale, nasale, per- ou transcutanée, rectale, perlinguale, oculaire, respiratoire, et par exemple les comprimés, ou dragées, les comprimés sublinguaux, les sachets, les capsules, les gélules, les suppositoires, et les ampoules injectables ou buvables.

Les exemples qui suivent illustrent l'invention sans en limiter la portée. Les composés sont, selon le cas, cités en noms chimiques ou en noms CTFA (International Cosmetic Ingrédient Dictionary and Handbook).

### Préparation A

### • Etape 1 : Arbutine peracétylée :

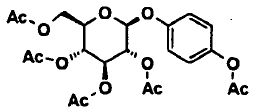

15g (55.1 mmol) d'Arbutine sont solubilisés dans 7 volumes de pyridine, puis 55ml (0.5 mol) d'Anhydride acétique sont ajoutés. Le milieu réactionnel est agité pendant 18h. Après concentration à moitié volume, le milieu est coulé lentement dans 100 volumes d'eau distillée sous agitation vigoureuse. Le solide blanc formé est filtré, lavé avec de l'eau et séché sous vide à 30°C pour conduire à 25.9g (53.7 mmol) de solide blanc..
Rf= 0.4 (toluène / acétate d'éthyle 1/1)

### • Etape 2 : Arbutine tétraacétylée :

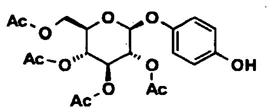

25.9g (53.7 mmol) d'Arbutine péracétylée sont solubilisés dans 10 volumes de méthanol et 10 volumes de tétrahydrofurane, sous flux d'azote et à l'abri de la lumière. Une pointe de spatule de carbonate de potassium est ajoutée et le milieu est agité pendant 1h30. Le suivi sur chromatographie en couche mince est régulier pour stopper la réaction avant une totale désacétylation. Après hydrolyse par 2 volumes de HCl 1M, le milieu est extrait par du dichlorométhane. Les phases organiques sont lavées par une solution saturée de NaCl, séchées sur MgSO4, filtrées et concentrées sous vide pour conduire à 18g (40.9 mmol) de solide blanc.
Rf=0.6 (toluène / acétate d'éthyle 1/1)

### Exemple 1

### Ester de 2-triméthylsilanyl-éthyle de l'acide (2E,4E,6E,8E)-3,7-diméthyl-9-{2,6,6-triméthyl-3-[4-((2S,3R,4S,5R,6R)3,4,5-triacétoxy-6-acétoxyméthyltétrahydro-pyran-2-yloxy)-phénoxy]-cyclohex-1-ényl}-nona-2,4,6,8-tétraènoique.

### • Etape 1 :

### Ester de 2-triméthylsilanyl-éthyle de l'acide (2E,4E,6E,8E)-3,7-diméthyl-9-(2,6,6-triméthyl-cyclohex-1-ényl)-nona-2,4,6,8-tétraènoique

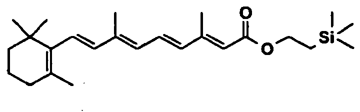

40g (0.13 mol) d'acide rétinoïque sont solubilisés dans 65 volumes d'acétate d'éthyle, sous flux d'azote et à l'abri de la lumière. A 0°C, 27.5g (0.13 mol) de dicyclohexylcarbodiimide, 19.3ml (0.136 mol) de triméthylsyliléthanol et 6.3g (0.05 mol) de diméthylaminopyridine sont ajoutés. Le milieu réactionnel est agité à température ambiante pendant 18h. Après filtration sur célite, le filtrat est concentré sous vide. 30g d'un produit brut sont purifiés sur colonne de silice, éluée par du toluène pour conduire à 22g (0.055 mol) d'un solide jaune.
Rf=0.8 (heptane / acétate d'éthyle 8/2)

### • Etape 2 :

### Ester de 2-triméthylsilanyl-éthyle de l'acide (2E,4E,6E,8E)-3,7-diméthyl-9-(2,6,6-triméthyl-3-oxo-cyclohex-1-ényl)-nona-2,4,6,8-tétraènoique.

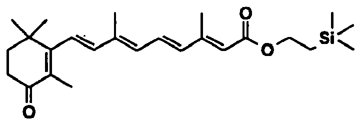

22g (55.0 mmol) du composé décrit à l'étape 1 sont solubilisés dans 40 volumes de dichlorométhane, sous flux d'azote et à l'abri de la lumière. 329g (3.78 mol) d'oxyde manganèse sont ajoutés et le mélange est agité pendant 24h. Le milieu est filtré sur célite, concentré sous vide. 25g de produit brut sont purifiés sur colonne de silice, éluée par un gradient heptane / acétate d'éthyle pour conduire à 14.3g (34.5 mmol) d'un solide orange.
Rf= 0.5 (heptane / acétate d'éthyle 7/3)

### • Etape 3 :

### Ester de 2-triméthylsilanyl-éthyle de l'acide (2E,4E,6E,8E)-9-(3-Hydroxy-2,6,6-triméthyl-cyclohex-1-ényl)-3,7-diméthyl-nona-2,4,6,8-tétraènoique.

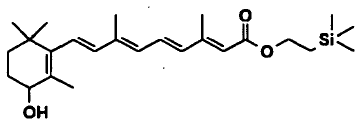

14.3g (34.5 mmol) décrit à l'étape 2 sont solubilisés dans 10 volumes de tétrahydrofurane, sous flux d'azote et à l'abri de la lumière. Le milieu est refroidi à -78°C et 29 ml (35.2 mmol) d'une solution de Dibal-H à 20% dans le toluène sont additionnés goutte-à-goutte en contrôlant la température à -78°C. Le milieu est agité pendant 5h. 20 volumes d'une solution saturée de sels de Rozen sont ajoutés lentement à -78°C. Le milieu est extrait à l'acétate d'éthyle. Les phases organiques sont lavées avec une solution saturée de NaCl, séchées sur MgSO₄, et concentrées sous vide. 16g de produit brut sont purifiés sur colonne de silice, éluée par un gradient heptane / acétate d'éthyle pour conduire à 10g (24.0 mmol) d'un solide jaune.
Rf= 0.4 (heptane / acétate d'éthyle 7/3)

### • Etape 4 :

### Ester de 2-triméthylsilanyl-éthyle de l'acide (2E,4E,6E,8E)-3,7-diméthyl-9-12,6,6-triméthyl-3-14-((2S,3R,4S,5R,6R)-3,4,5-triacétoxy-6-acétoxyméthyltétrahydro-pyran-2-yloxy)-phénoxy]-cyclohex-1-ényl}-nona-2,4,6,8-tétraènoique.

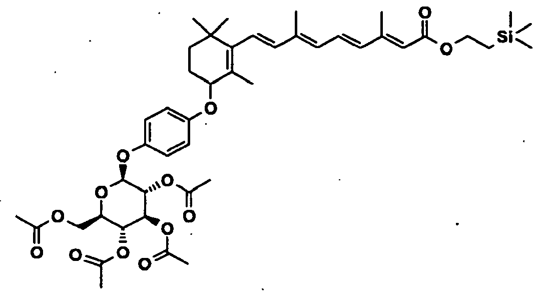

11.8g (26.8 mmol) d'Arbutine tetraacétylée (voir préparation A) sont solubilisés dans 10 volumes de tétrahydrofurane et 3 volumes de toluène, sous flux d'azote. A -5°C, une solution fraîchement préparée de 5.2ml (29.5 mmol) de diisopropyl azodicarboxylate avec 10.4g (40.2 mmol) de triphénylphosphine dans 10 volumes de toluène, est additionnée goutte-à-goutte. Le milieu est agité à 0°C pendant 10 minutes, puis, à l'abri de la lumière, 5g (12.0 mmol) du composé décrit à l'étape 3 dans 10 volumes de toluène sont additionnés lentement. Le mélange est agité pendant 20h. Le milieu est concentré sous vide et le résidu est purifié sur colonne de silice, éluée par un gradient heptane / acétate d'éthyle pour conduire à 3.0g (3.6 mmol) d'un solide jaune.
Rf= 0.3 (heptane / acétate d'éthyle 7/3)
Spectrométrie de masse : [MNa⁺] 861 ([MH+] calculé 839)

### Exemple 2

### Ester de 2-triméthylsilanyl-éthyle de l'acide (2E,4E,6E,8E)-3,7-diméthyl-9-{2,6,6-triméthyl-3-[4-((2S,3R,4S,5S,6R)-3,4,5-trihydroxy-6-hydroxyméthyltétrahydro-pyran-2-yloxy)-phénoxy]-cyclohex-1-ényl}-nona-2,4,6,8-tétraènoique.

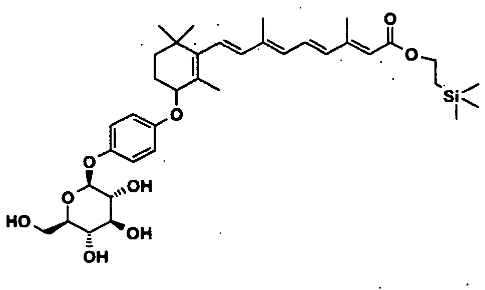

0.5g (0.6 mmol) de composé de l'exemple 1 sont solubilisés, à l'abri de la lumière, dans 4 volumes de tétrahydrofurane. 3 volumes de méthanol sont alors ajoutés ainsi qu'une pointe de spatule de carbonate de potassium. Le mélange est agité pendant 6h. Après hydrolyse à l'eau distillée, le milieu est concentré, puis extrait au dichlorométhane. Les phases organiques sont lavées avec une solution saturée de NaCl, séchées sur MgSO₄ et concentrées sous vide pour conduire à 30 mg (0.04 mmol) d'un solide jaune pâle.
Rf= 0.1 (heptane / acétate d'éthyle 7/3)
Spectrométrie de masse : [MNa⁺] 693 ([MH+] calculé 671)

### Exemple 3

### Acide (2E,4E,6E,8E)-3,7-diméthyl-9-{2,6,6-triméthyl-3-[4-((2S,3R,4S,5S,6R)-3,4,5-trihydroxy-6-hydroxyméthyl-tétrahydro-pyran-2-yloxy)-phénoxy]-cyclohex-1-ényl}-nona-2,4,6,8-tétraènoique.

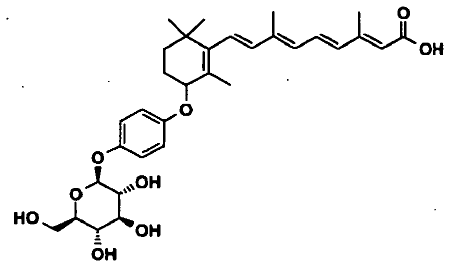

0.5g (0.6 mmol) de composé de l'exemple 1 sont mis en suspension dans 10 volumes d'éthanol, à l'abri de la lumière. 0.3ml (0.6 mmol) d'une solution de soude 2M sont ajoutés au milieu réactionnel. Le mélange est agité pendant 7h. 1.8ml de soude 2M sont à nouveau ajoutés. Après hydrolyse acide (HCl 6N), jusqu'à pH =5.8, le milieu est filtré et le résidu solide séché sous vide pour conduire à 0.07g (0.12 mmol) d'un solide jaune pâle.
Rf= 0.1 dichlorométhane / méthanol 9/1)
Spectrométrie de masse : [MNa⁺] 593 ([MH+] calculé 571)

### Exemple 4

### Ester tert-butylique de l'acide (2E,4E,6E,8E)-9-[3-(4-Méthoxy-phénoxy)-2,6,6-triméthyl-cyclohex-1-ényl]-3,7-diméthyl-nona-2,4,6,8-tétraènoique.

### • Etape 1 :

### Ester tert-butylique de l'acide (2E,4E,6E,8E)-3,7-diméthyl-9-(2,6,6-triméthylcyclohex-1-ényl)-nona-2,4,6,8-tétraènoique.

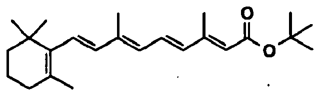

9.1g (30.3 mmol) d'acide rétinoïque sont solubilisés dans 10 volumes de dichlorométhane. A température ambiante, sous flux d'azote et à l'abri de la lumière, 14g (69.6 mmol) de 2-*tert*-butyl-1,3-diisopropyl-isourée sont ajoutés. Le milieu est agité pendant 18h. Le suivi sur chromatographie en couche mince révèle la fin de la réaction (révélation UV). Les sels d'urée sont alors filtrés, le filtrat est concentré à l'abri de la lumière et purifié sur colonne de silice avec, pour éluant, heptane / acétate d'éthyle 9/1 à l'abri de la lumière pour conduire à 10.4g (29.2 mmol) d'une huile pâteuse.
Rf = 0.4 (heptane / acétate d'éthyle 8/2)

### • Etape 2 :

### Ester tert-butylique de l'acide (2E,4E,6E,8E)-3,7-diméthyl-9-(2,6,6-triméthyl3-oxo-cyclohex-1-ényl)-nona-2,4,6,8-tétraènoique.

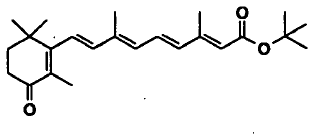

10.4g (29.2 mmol) du composé de l'étape 1 sont solubilisés dans 40 volumes de dichlorométhane, sous flux d'azote et à l'abri de la lumière. 114g (131 mmol) d'oxyde manganèse sont ajoutés et le mélange est agité pendant 72h. Le milieu est filtré sur célite, concentré sous vide et purifié sur colonne de silice par un mélange heptane / acétate d'éthyle 9/1. Après cristallisation, 10g (27.0 mmol) de solide orange sont obtenus.
Rf= 0.5 (heptane / acétate d'éthyle 7/3)

### • Etape 3 :

### Ester tert-butylique de l'acide (2E,4E,6E,8E)-9-(3-Hydroxy-2,6,6-triméthylcyclohex-1-ényl)-3,7-diméthyl-nona-2,4,6,8-tétraènoique.

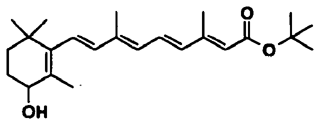

10g (27.0 mmol) du composé de l'étape 2 sont solubilisés dans 10 volumes de tétrahydrofurane, sous flux d'azote et à l'abri de la lumière. Le milieu est refroidi à -78°C et 23 ml (27.5 mmol) d'une solution de Dibal-H à 20% dans le toluène sont additionnés goutte-à-goutte en contrôlant la température à -78°C. Le milieu est agité pendant 3h. A -60°C, 20 volumes d'une solution saturée de sels de Rozen sont ajoutés. Le milieu est extrait au dichlorométhane. Les phases organiques sont lavées avec une solution saturée de NaCl, séchées sur MgSO₄, et concentrées sous vide. 15g de produit brut sont purifiés sur colonne de silice, éluée par un gradient heptane / acétate d'éthyle pour conduire à 6.8g (18.3 mmol) d'une huile jaune pâteuse.
Rf = 0.3 (heptane / acétate d'éthyle 7/3)

### • Etape 4 :

### Ester tert-butylique de l'acide (2E,4E,6E,8E)-9-[3-(4-Méthoxy-phénoxy)-2,6,6-triméthyl-cyclohex-1-ényl]-3,7-diméthyl-nona-2,4,6,8-tétraènoique.

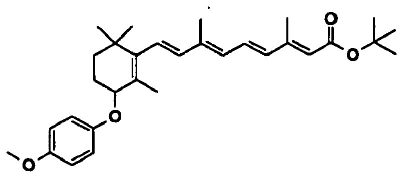

0.67g (5.2 mmol) de 4-Méthoxyphénol sont solubilisés dans 20 volumes de toluène, sous flux d'azote. A -5°C, une solution fraîchement préparée de 0.58ml (28.6mmol) Diisopropyl azodicarboxylate avec 1.1g (38.9mmol) de triphénylphosphine dans 40 volumes de toluène, est additionnée goutte à goutte. Le milieu est agité à 0°C pendant 10 minutes, puis, à l'abri de la lumière, 0.97g (26.0 mmol) de composé de l'étape 3 dans 20 volumes de toluène sont additionnés lentement. Le mélange est agité pendant 20h. Le milieu est concentré sous vide et le résidu est purifié sur colonne de silice, éluée par un système heptane / acétate d'éthyle 98/2 pour conduire à 0.5g d'une huile jaune.
Rf= 0.7 (heptane / acétate d'éthyle 8/2)
Spectrométrie de masse : [MNa⁺] 501 ([MH⁺] calculé 479)

### Exemple 5

### (2E,4E,6E,8E)-9-[3-(4-Méthoxy-phénoxy)-2,6,6-triméthyl-cyclohex-1-ényl]-3,7-diméthyl-nona-2,4,6,8-tétraénal

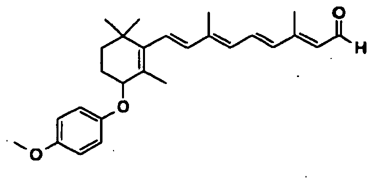

0.5g (1.04 mmol) de composé de l'exemple 4 sont solubilisés dans 10 volumes de toluène, sous flux d'azote et à l'abri de la lumière. Le milieu est refroidi à -78°C et 0.89 ml (1.07 mmol) d'une solution de Dibal-H à 20% dans le toluène sont additionnés goutte-à-goutte en contrôlant la température à -78°C. Le milieu est agité pendant 10 minutes. A -78°C, 10 volumes d'une solution saturée de sels de Rozen sont ajoutés. Le milieu est extrait au toluène. Les phases organiques sont lavées avec une solution saturée de NaCl, séchées sur MgSO₄, et concentrées sous vide. 0.8g de produit brut sont purifiés sur colonne de silice, éluée par un gradient heptane / acétate d'éthyle. Les cristaux jaunes obtenus sont lavés dans le pentane pour conduire à 150mg (0.37 mmol) d'un solide jaune.
Rf= 0.2 (heptane / acétate d'éthyle 8/2)
Spectrométrie de masse : [MNa⁺] 429 ([MH⁺] calculé 407)

### Exempte 6

### Ester tert-butylique de l'acide (2E,4E,6E,8E)-9-[3-(4-Hydroxy-phénoxy)-2,6,6-triméthyl-cyclohex-1-ényl]-3,7-diméthyl-nona-2,4,6,8-tétraènoique.

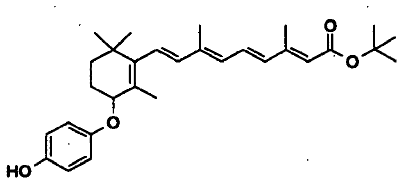

1.1g (10.06mmol) d'Hydroquinone sont solubilisés dans 40 volumes de Tétrahydrofurane sous flux d'azote. A -5°C, une solution fraîchement préparée de 0.87ml (4.42mmol) Diisopropyl azodicarboxylate avec 1.6g (6.04mmol) de Triphénylphosphine dans 30 volumes de Toluène et 30 volumes de Tétrahydrofurane, est additionnée goutte-à-goutte. Le milieu est agité à 0°C pendant 10 minutes, puis, à l'abri de la lumière, 1.5g (4.03 mmol) de composé de l'exemple 4 dans 20 volumes de Toluène sont additionnés lentement. Le mélange est agité pendant 20h. Le milieu est concentré sous vide et le résidu est purifié sur colonne de silice, éluée par un système heptane / acétate d'éthyle 8/2 pour conduire à 0.6g d'un solide jaune.
Rf= 0.6 (heptane / acétate d'éthyle 7/3)
Spectrométrie de masse : [MNa⁺] 487 ([MH⁺] calculé 465)

### Exemple 7

### (2E,4E,6E,8E)-9-[3-(4-Hydroxy-phenoxy)-2,6,6-trimethyl-cyclohex-1-enyl]-3,7-dimethyl-nona-2,4,6,8-tetraenal

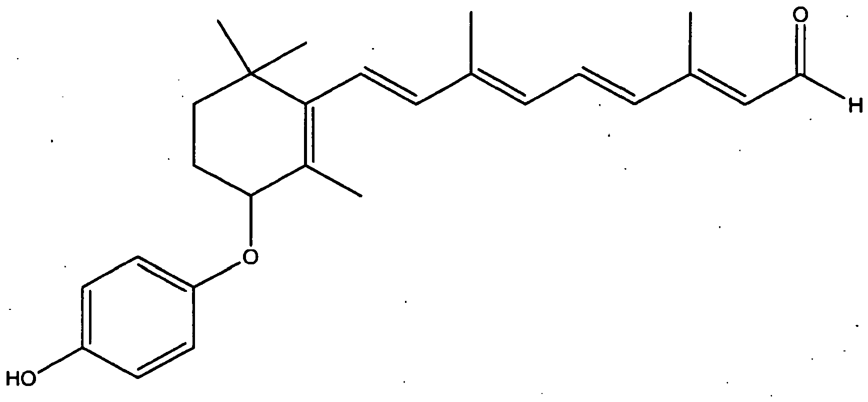

6.9g (31.0 mmol) d'hydroquinone mono-protégée par un groupement tertbutyldimethylsilyle sont solubilisés dans 10 volumes de Toluène sous flux d'azote. A -5°C, une solution fraîchement préparée de 7.9 ml (40.3 mmol) Diisopropyl azodicarboxylate avec 12.2g (46.5 mmol) de Triphénylphosphine dans 10 volumes de Toluène, est additionnée goutte-à-goutte. Le milieu est agité à 0°C pendant 10 minutes, puis, à l'abri de la lumière, 6g (2.68 mmol) de l'ester tert-butylique de l'acide (2E,4E,6E,8E)-9-(3-Hydroxy-2,6,6-triméthyl-cyclohex-1-ényl)-3,7-diméthyl-nona-2,4,6,8-tétraènoique tel qu'obtenu à l'étape 3 de l'exemple 4dans 10 volumes de Toluène sont additionnés lentement. Le mélange est agité pendant 20h. Le milieu est concentré sous vide et le résidu est purifié sur colonne de silice, éluée par un gradient heptane / éther isopropylique. 5.16g (8.91 mmol) d'un solide jaune sont obtenus (55%).

### Déprotection et obtention de la fonction aldéhyde :

1.32g (2.28 mmol) du composé précédent sont mis en solution dans 10 volumes de toluène, sous flux d'azote, à l'abri de la lumière. Le milieu est refroidi à -80°C et 2.46 ml d'une solution de Dibal-H à 20% dans le toluène sont additionnés lentement. Le milieu est agité pendant 20 min puis 20 volumes d'une solution saturée de sels de Rozen sont ajoutés à -78°C. Après une agitation vigoureuse, le milieu est extrait au toluène. Les phases organiques sont lavées par une solution saturée dé NaCl, séchées sur MgSO₄, et concentrées sous vide. Le mélange brut est purifié sur colonne de silice, conditionnée et éluée par un gradient heptane / ether isopropylique. 0.58g (1.18 mmol) d'un solide jaune sont obtenus (51% de rendement).

1.87g (3.69 mmol) du dérivé précédent sont mis en solution dans 10 volumes de Tétrahydrofurane, à l'abri de la lumière. A 0°C, 5.2 ml (5.17 mmol) d'une solution 1M de Tétrabutylammonium fluoride sont additionnés goutte à goutte. Le milieu est agité pendant 20min, puis est coulé sur une solution saturée de NH₄Cl. Après extraction à l'éther isopropylique, les phases organiques sont lavées par une solution saturée de NaCl, puis séchées sur Na₂SO₄ et concentrées sous vide. Le mélange brut est purifié sur colonne de silice, conditionnée dans l'heptane et éluée par un gradient heptane / acétate d'éthyle. 1g (2.55 mmol) d'un solide rouge-orangé sous obtenus (69% de rendement)

### Exemple 8

### 9-[(3-trans décènoate)-2,6,6 triméthyl-cyclohex-1-ényl]-3,7-diméthylnona-2,4,6,8 tétraènal

### • Etape 1 :

### 9-(2,6,6-triméthyl-cyclohex-1-ényl)-3,7-diméthyl-nona-2,4,6,8-tétraènal

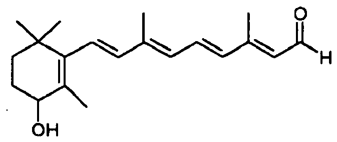

2g (5.37 mmol) de l'ester tert butylique de l'acide hydroxy rétinoïque, préparé comme à l'étape 3 dé l'exemple 4 sont mis en solution dans 10 volumes de toluène, sous flux d'azote et à l'abri de la lumière. Le milieu est refroidi à -78°C, puis 4.57ml (5.48 mmol) d'une solution de Dibal-H à 20% dans le toluène sont ajoutés goutte-à-goutte. Après 3h d'agitation à -78°C, 20 volumes d'une solution saturée de sels de Rozen sont ajoutés. Le milieu est extrait au toluène, les phases organiques sont lavées avec une solution saturée de NaCl, séchées sur MgSO₄, filtrées et concentrées sous vide. Le produit brut obtenu est purifié sur colonne de silice, éluée par un gradient Heptane, Heptane / Acétate d'éthyle 9/1.180mg d'un solide jaune est obtenu (20%).
Rf= 0.2 (heptane / acétate d'éthyle 7/3)
Spectrométrie de masse : [M+Na⁺] = 323 ([MH⁺] calculé 300)

### • Etape 2 :

### 9-[(3-trans décènoate)-2,6,6 triméthyl-cyclohex-1-ényl]-3,7-diméthyl-nona2,4,6,8 tétraènal

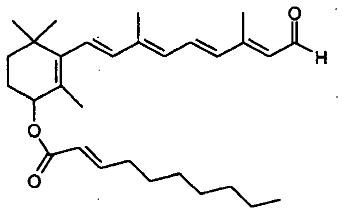

0.3g (1 mmol) de composé de l'étape 1 sont mis en solution dans 30 volumes de dichlorométhane, à 0°C. 0.14g (1.15eq) de diméthylaminopyridine sont ajoutés ainsi que 1.1eq d'acide trans-2-décénoïque. 0.24g de dicyclohexylcarbodiimide sont coulés et le mélange réactionnel est agité 24h à température ambiante. Le milieu est filtré sur célite pour éliminer les sels d'urée, et le filtrat est concentré. Une purification par chromatographie sur gel de silice conduit au produit attendu.
Rf= 0.7 (heptane / acétate d'éthyle 7/3)
Spectrométrie de masse : [MNa⁺] 475 ([MH⁺] calculé 452)

### Exemple 9

### 9-[(3-linoléate)-2,6,6-triméthyl-cyclohex-1-ényl]-3,7-diméthyl-nona-2,4,6,8 tétraènal

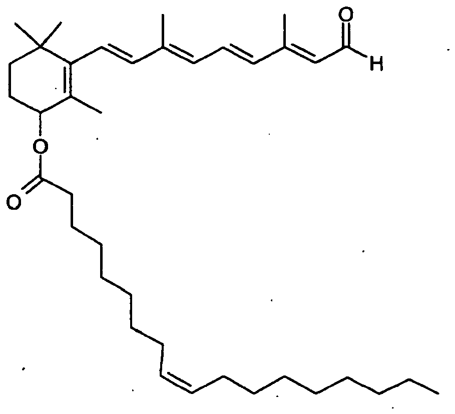

0.3g (1 mmol) du composé de l'exemple 7, étape 1 sont mis en solution dans 30 volumes de dichloroéthane. 0.09ml (1eq) de pyridine sont ajoutés puis lentement 1.1eq de dérivé chlorure oléyle est coulé au milieu réactionnel. Le milieu est agité pendant 7h à température ambiante. Le milieu est hydrolysé, extrait au dichloroéthane. Les phases organiques sont lavées par une solution saturée de NaCl, puis par une solution HCl 0.1M et enfin par une solution saturée de NaCl. Après séchage sur Na₂SO₄ et filtration, les phases organiques sont concentrées sous vide. Le produit brut obtenu est purifié par chromatographie sur silice pour conduire au composé attendu.
Rf = 0.7 (heptane / acétate d'éthyle 7/3)
Spectrométrie de masse : [MNa⁺] 587 ([MH⁺] calculé 564)

### Exemple 10

### 9-[(3-linoléate)-2,6,6-triméthyl-cyclohex-1-ényl]-3,7-diméthyl-nona-2,4,6,8-tétraènal

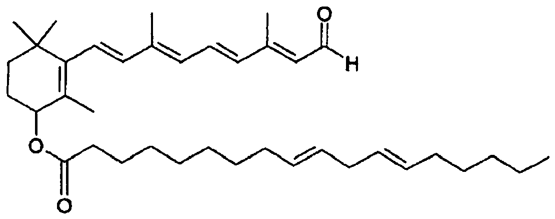

Le produit attendu est obtenu selon un procédé identique à celui décrit dans l'exemple 8, en remplaçant le chlorure d'oléyle par le chlorure de linoléyle.

### Exemple 11

### 9-[(3-lipoate)-2,6,6-triméthyl-cyclohex-1-ényl]-3,7-diméthyl-nona-2,4,6,8-tétraènal

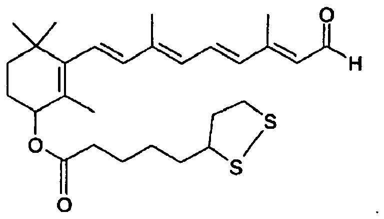

Le produit attendu est obtenu selon le procédé décrit dans l'exemple 8 en remplaçant le chlorure d'oléyle par le chlorure de lipoyle.

### Exemple 12

### 9-[{3-(8-hydroxy-5-méthyl-2-octènoate)}-2,6,6-triméthyl-cyclohex-1-ényl]-3,7-diméthyl-nona-2,4,6,8-tétraènal

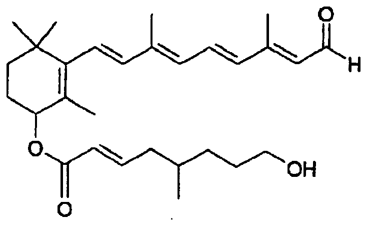

Le produit attendu est obtenu selon le procédé décrit dans l'exemple 8 en remplaçant le chlorure d'oléyle par le chlorure de l'acide 8-hydroxy-5-méthyl-2-octénoïque.

### Exemple 13

### 9-[(3-tetraacétyl glucose)-2,6,6-triméthyl-cyclohex-1-ényl]-3,7-diméthyl-nona2,4,6,8-tétraènal

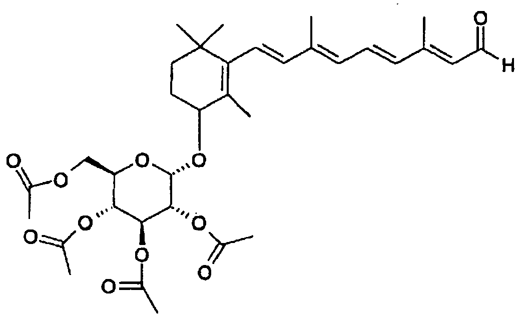

8ml (2.5 eq) d'une solution de ZnCl₂ 1.0M dans l'éther sont dilués dans 2 volumes de toluène et 2 volumes d'acétonitrile, en présence de 0.7g de tamis moléculaire 4Å. Après une agitation de 10 min, le milieu réactionnel est plongé dans un bain à 90°C, et 0.21g (0.70 mmol, 1 eq) du composé de l'exemple 7, étape 1, sont additionnés. Après 10min d'agitation, 0.32g (0.77 mmol, 1.1eq) de 2,3,4,6-tetra-O-acetyl-alpha-D-glucopyranosylbromide et 0.25ml (1.75 mol, 2.5eq) de triéthylamine sont ajoutés. Le milieu réactionnel est chauffé pendant une nuit à 80°C. A température ambiante, le milieu est filtré et le filtrat est mis en agitation avec une solution saturée de NaHCO₃. Après extraction et lavage par une solution saturée de NaCl, les phases organiques sont concentrées sous vide. Le produit brut est purifié par chromatographie sur silice pour conduire au composé attendu.
Rf= 0.7 (heptane / acétate d'éthyle 7/3)
Spectrométrie de masse : [M+Na⁺] = 653 ([M+H]⁺ calculé 630)

### Exemple 14

### Ester 3-((1E,E,5E,7E)-3,7-dimethyl-9-oxo-nona-1,3,5,7-tetraenyl)-2,4,4-trimethyl-cyclohex-2-enyl de l'acide (9Z,12Z,15Z)-Octadeca-9,12,15-trienoique

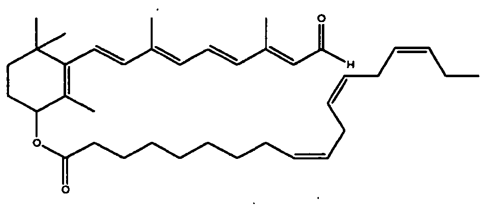

Dans un tricol sec sous flux d'azote, à l'abri de la lumière, 0.25g (0.83mmol) d'Hydroxy-rétinal sont mis en solution dans 35 volumes de Dichlorométhane. 0.28g (1.2eq) d'acide linolénique sont ajoutés au milieu ainsi que 0.13g (1eq) de diméthylaminopyridine. Le Dicyclohexylcarbodiimide (0.20g, 1eq) dans 5 volumes de Dichlorométhane est lentement coulé. Le milieu réactionnel est agité pendant une nuit à température ambiante. Un suivi CCM permet de contrôler la fin de la réaction. Le milieu est alors filtré et concentré sous vide. Le résidu est purifié sur colonne de silice, à l'abri de la lumière, éluée par un gradient heptane / acétate d'éthyle, pour conduire à un solide jaune (0.40g, soit un rendement près de 85%).
CCM (Heptane/ acétate d'éthyle 7/3) : R_{f} = 0.8
Spectrométrie de masse : [M+Na]⁺ = 583 (calculée 560)

### Exemple 15

### Ester 3-((1E,3E,5E,7E)-3,7-dimethyl-9-oxo-nona-1,3,5,7-tetraenyl)-2,4,4-trimethyl-cyclohex-2-enyl de l'acide (9Z,12Z)-Octadeca-9,12-dienoique

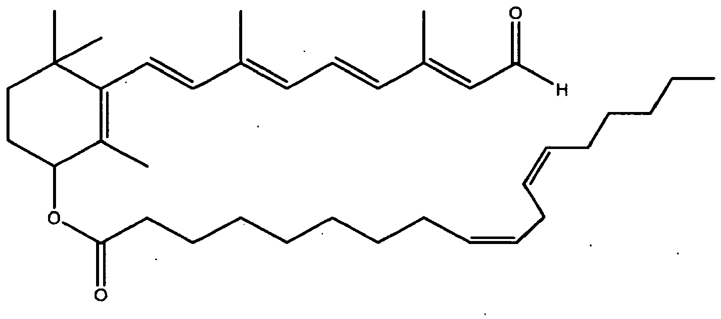

Les mêmes conditions opératoires qu'à l'exemple 13 sont appliquées à l'Hydroxyrétinal et à l'acide linoléique; pour réaliser l'estérification, sur une échelle de 250mg avec un rendement de 34%.

### Exemple 16

### Ester 3-((1E,3E,5E,7E)-3,7-dimethyl-9-oxo-nona-1,3,5,7-tetraenyl)-2,4,4-trimethyl-cyclohex-2-enyl de l'acide (E)-14-Hydroxy-tetradec-2-enoique

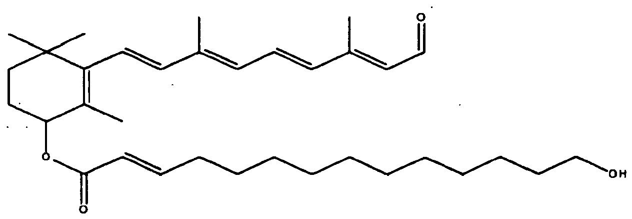

Les mêmes conditions opératoires de protection - estérification - déprotection sont appliquées à l'Hydroxyrétinal et à l'acide (E)-14-tetradec-2-enoïque (préalablement synthétisé), sur une échelle de 500mg avec un rendement global de 15%.
CCM (Heptane/ acétate d'éthyle 7/3) : R_{f}= 0.2
Spectrométrie de masse : [M+Na]⁺ = 547 (calculée 524)

### Exemple 17

### Ester 3-((1E,3E,5E,7E)-3,7-dimethyl-9-oxo-nona-1,3,5,7-tetraenyl)-2,4,4-trimethyl-cyclohex-2-enyl de l'acide (E)-10-Acetoxy-dec-2-enoique

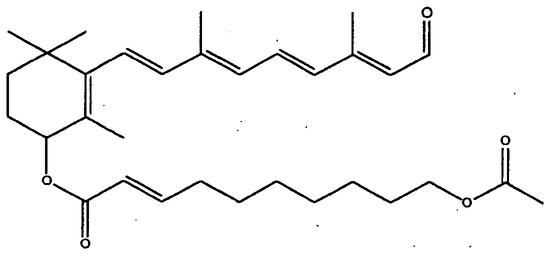

Dans un tricol sous flux d'azote, 1g (5.3mmol) d'acide (E)-10-Hydroxy-dec-2-enoïque est solubilisé dans 10 volumes de pyridine. 546µl (1.1eq) d'anhydride acétique est coulé. Le milieu réactionnel est agité à température ambiante toute une nuit. Un contrôle CCM permet de valider la fin de la réaction. Le milieu est concentré sous vide. Le résidu est repris par une solution de saumure et de l'acétate d'éthyle. Le mélange est extrait à l'acétate d'éthyle et les phases organiques rassemblées sont lavées par une solution d'acide chlorhydrique 1M, puis par une solution saturée de NaCl. Elles sont alors séchées sur MgSO₄, filtrées et concentrées sous vide, pour conduire à une huile marron. Ce produit brut est purifié par colonne de silice, éluée par un gradient heptane / acétate d'éthyle. 850mg d'une huile jaune sont obtenus (70% de rendement).

Dans un tricol sec sous flux d'azote, 0.25g (0.83mmol, 1.2eq) de cristaux jaunes d'Hydroxyrétinal sont mis en solution dans 40 volumes de dichlorométhane. L'hydroxyacide protégé sous forme acétate (0.25g, 1eq) est dilué dans 40 volumes de Dichlorométhane et est coulé au milieu réactionnel. 0.15g (1eq) de Diméthylaminopyridine sont additionnés puis le Dicyclohexylcarbodiimide (0.38g, 1.2eq) dissous dans 20 volumes de Dichlorométhane est coulé lentement au milieu réactionnel. Le mélange est agité à l'abri de la lumière pendant une nuit. Un suivi CCM permet de contrôler la fin de la réaction. Le milieu réactionnel est filtré et le filtrat est concentré. Le résidu obtenu est purifié sur colonne de silice, éluée par un gradient heptane / acétate d'éthyle, à l'abri de la lumière. 0.28g d'une huile jaune est alors obtenue (soit un rendement de 65%).
CCM (Heptane/ acétate d'éthyle 7/3) : R_{f}= 0.4

### Exemple 18 :

### Ester 3-((1E,3E,5E,7E,3,7-dimethyl-9-oxo-nona-1,3,5,7-tetraenyl)-2,4,4-trimethyl-cyclohex-2-enyl de l'acide (E)-10-Hydroxy-dec-2-enoique

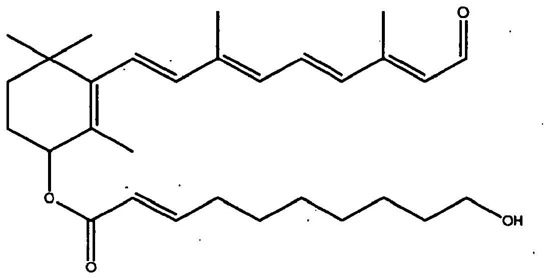

On procède comme dans l'exemple 18 puis dans un ballon, 0.2g d'huile précédente est diluée dans 40 volumes de méthanol. 467µl d'une solution fraîchement préparée de KHCO₃ et K₂CO₃ (respectivement 70mg et 50mg dans 1ml de méthanol) sont additionnés. Le milieu est agité à température ambiante, à l'abri de la lumière, pendant 4h30. Un suivi CCM permet de contrôler la fin de la déprotection. Le milieu est hydrolysé par ajout d'eau. Il est ensuite extrait au dichlorométhane. Les phases organiques sont rassemblées, lavées par une solution saturée de NaCl, séchées sur MgSO₄, filtrées et concentrées. Le résidu est purifié sur colonne de silice, éluée par un gradient heptane / acétate d'éthyle, à l'abri de la lumière. 0.11 g d'un solide jaune est alors obtenu, ce qui conduit à un rendement de 60%.
CCM (Heptane/ acétate d'éthyle 7/3) : R_{f}= 0.2
Spectrométrie de masse : [M+Na]⁺ = 491 (calculée 468)

### Exemple 19

### Bis ester 3-((1E,3E,5E,7E,3,7-dimethyl-9-oxo-nona-1,3,5,7-tetraenyl)-2,4,4-trimethyl-cyclohex-2-enyl et (R)-2,8-dimethyl-2-((4R, 8R)-4,8,12-trimethyltridecyl)-1-benzopyran-6-yl de l'acide heptanedioique

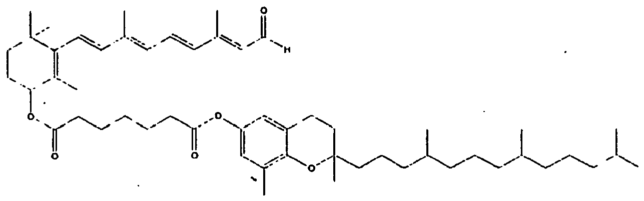

Dans un tricol sec, sous flux d'azote, 0.8g (0.95eq) d'acide pimélique sont mis en solution dans 20 volumes de dichlorométhane. 1.1g (1eq) de Dicyclohexylcarbodiimide et 0.63g (1eq) de Diméthylaminopyridine sont ajoutés. Après 5 min d'agitation à température ambiante 2.1g (5.2mmol) dé (+)-deltatocopherol dans 10 volumes de dichlorométhane sont coulés lentement. Le milieu réactionnel est agité 6h. Un suivi CCM permet de contrôler la fin de la réaction. Le milieu est alors filtré et concentré. Le résidu est purifié sur colonne de silice, éluée par un gradient heptane / acétate d'éthyle. 0.35g d'une huile sont alors obtenus (rendement 12%).

Dans un tricol sec sous flux d'azote, à l'abri de la lumière, 0.17g (0.57 mmol) d'Hydroxy-retinaldehyde sont mis en solution dans 30 volumes de Dichlorométhane, à 0°C. 0.08g (1.2eq) de Diméthylaminopyridine sont ajoutés ainsi que 0.35g (1.15eq) du dérivé tocopherol-espaceur en C7 obtenu précédemment. 0.17g (1.4eq) de Dicyclohexylcarbodiimide dans 20 volumes de Dichlorométhane sont coulés au milieu, à 0°C, et l'agitation est réalisée pendant 24h à température ambiante. Un suivi CCM permet de contrôler la fin de la réaction. Le milieu est filtré sur célite pour éliminer les sels d'urée et le filtrat est concentré. Le produit brut est purifié par chromatographie sur silice, à l'abri de la lumière, pour conduire à une huile jaune-orangé (0.28g, soit un rendement de 60%).
CCM (Heptane/ acétate d'éthyle 7/3) : R_{f}= 0.5
Spectrométrie de masse : [M+Na]⁺ = 849 (calculée 827)

### Exemple 20:

### Bis ester 3-((1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R,8R)-4,8,12-trimethyltridecyl)-1-benzopyran-6-yl de l'acide nonanedioique

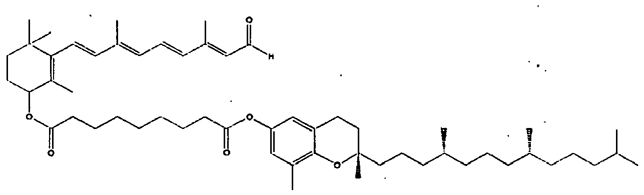

Les mêmes conditions opératoires que dans l'exemple précédent sont appliquées au (+)-delta-Tocopherol et à l'acide azélaïque. Ce dérivé est ensuite couplé à l'Hydroxyrétinal par estérification, sur une échelle opératoire de 250mg avec un rendement global de 11%.
CCM (Heptane/ acétate d'éthyle 7/3) : R_{f}= 0.6
Spectrométrie de masse : [M+Na]⁺ = 877 (calculée 854)

### Exemple 21 :

### Bis ester 3- (1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R,8R)-4,8,12-trimethyltridecyl)-1-benzopyran-6-yl de l'acide succinique

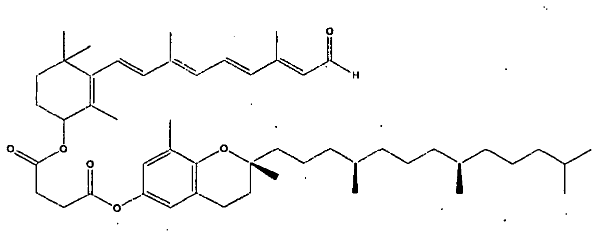

Les mêmes conditions opératoires que précédemment sont appliquées au (+)-delta-Tocopherol et à l'acide succinique. Ce dérivé est ensuite couplé à l'Hydroxyrétinal par estérification, sur une échelle opératoire de 200mg avec un rendement global de 3%.
CCM (Heptane/ acétate d'éthyle 7/3) : R_{f}= 0.5

### Exemple 22 :

### Bis ester 3-((1E,3E,5E,7E)-3,7-dimethyl-9-oxo-nona-1,3,5,7-tetraenyl)-2,4,4-trimethyl-cyclohex-2-enyl et (R)-2,8-dimethyl-2-((4R,8R)-4,8,12-trimethyltridecyl)-1-benzopyran-6-yl de l'acide pentanedioique

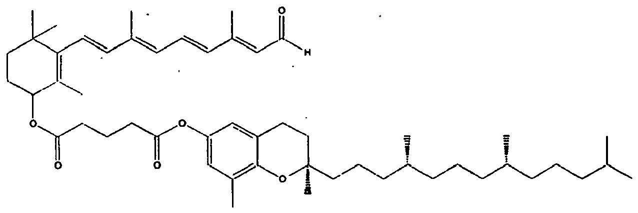

Les mêmes conditions opératoires que précédemment sont appliquées au (+)-delta-Tocopherol et à l'acide glutarique. Ce dérivé est ensuite couplé à l'Hydroxyrétinal par estérification, sur une échelle opératoire de 250mg avec un rendement global de 21%.
CCM (Heptane/ acétate d'éthyle 7/3) : R_{f}= 0.6

### Exemple 23 :

### Composition blanchissante :

| **Composition (émulsion H/E)** | **Quantité (g)** |
|---|---|
| Composé de l'exemple 3 | 0,1 |
| Vaseline | 8 |
| Glycérine 99.5% | 15 |
| Stéarate glyceryl SG | 5 |
| Stearique acide | 3 |
| Paraffine liquide 352 | 4 |
| Cyclopentasiloxane | 3 |
| Macrogol 600 | 5 |
| Ethanolamine (tri) | 0,5 |
| Paraben | 0,4 |
| Eau purifiée | QSP 100 |

### Lotion blanchissante :

| **Composition** | **Quantité (g)** |
|---|---|
| Composé de l'exemple 3 | 0,1 |
| Betaine cocamidopropyl B4F | 2 |
| Macrogol 600 | 4 |
| Phenylethyl alcool | 0,5 |
| Ethanolamine (tri) | 0,04 |
| Eau puri fiée | QSP 100 |

## Revendications

1. Composé de formule (I) : 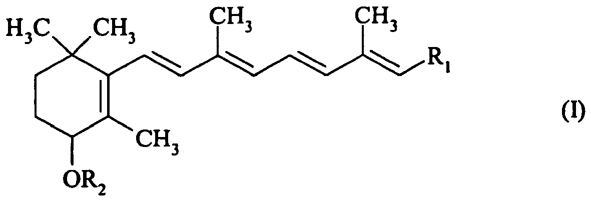 dans laquelle
R₁ représente un groupement R'₁ ou -A-R'₁, dans lesquels R'₁ est choisi parmi - COOH, -COOR₃, -CONH₂, -CONHR₃, -CONR₃R₄, -CHO, -CH₂OH, -CH₂OR₅, et A représente un groupement alkylène C₁-C₁₆ linéaire ou ramifié, alkénylèneC₂-C₁₆ linéaire ou ramifié, ou alkynylène C₂-C₁₆ linéaire ou ramifié,
R₂ représente :
• un groupement aryle éventuellement substitué ou hétéroaryle éventuellement substitué,
• un résidu osidique ou ,
• un résidu d'acide gras éventuellement ramifié et/ou substitué de préférence en bout de chaîne,
• un groupe -OC -(CH₂)ₙ - CO -tocophéryle (alpha, bêta ou gamma ou delta), avec 2≤ n ≤ 10,
• un groupe -R'₂-O-R₆, dans lequel R'₂ est un groupement arylène éventuellement substitué, et R₆ représente un atome d'hydrogène, un groupement alkyle C₁-C₆ linéaire ou ramifié, ou un résidu osidique, R₃ et R₄ représentent indépendamment un radical alkyle C₁-C₁₆ linéaire ou ramifié éventuellement substitué, alkényle C₂-C₁₆ linéaire ou ramifié éventuellement substitué, ou alkynyle C₂-C₁₆ linéaire ou ramifié éventuellement substitué,
R₅ représente un radical alkyle C₁-C₁₆ linéaire ou ramifié éventuellement substitué, alkényle C₂-C₁₆ linéaire ou ramifié éventuellement substitué, alkynyle C₂-C₁₆ linéaire ou ramifié, ou acyle C₂-C₁₆ linéaire ou ramifié,
leurs énantiomères, diastéréoisomères, ainsi que leurs éventuels sels d'addition à un acide ou à une base physiologiquement acceptable.

2. Composé de formule (I) selon la revendication 1, **caractérisé en ce que** R₁ représente un groupement choisi parmi -COOH, -COOR₃, -CHO, -CH₂OH, -CH₂OR₅, dans lesquels R₃ et R₅ sont tels que définis dans la revendication 1.

3. Composé de formule (I) selon la revendication 1, **caractérisé en ce que** A représente un groupement méthylène.

4. Composé de formule (I) selon l'une des revendications 1 à 3, **caractérisé en ce que** R₂ représente un groupe -R'₂-O-R₆, dans lequel R'₂ est un groupement arylène éventuellement substitué et R₆ représente un atome d'hydrogène, un groupement alkyle C₁-C₆ linéaire ou ramifié, ou un résidu osidique.

5. Composé de formule (I) selon la revendication 4, **caractérisé en ce que** R'₂ représente un groupement phénylène, et R₆ représente un résidu de sucre choisi parmi glucose, galactose, fructose, mannose, fucose et rhamnose,

6. Composé de formule (I) selon la revendication 1, **caractérisé en ce qu'**il est choisi parmi:
- l'acide (2E,4E,6E,8E)-3,7-Diméthyl-9-{2,6,6-triméthyl-3-[4-((2S,3R,4S,5S,6R)-3,4,5-trihydroxy-6-hydroxyméthyl-tétrahydro-pyran-2-yloxy)-phénoxy]-cyclohex-1-ényl}-nona-2,4,6,8-tétraénoique,
- l'ester tert-butylique de l'acide (2E,4E,6E,8E)-9-[3-(4-Méthoxy-phénoxy)-2,6,6-triméthyl-cyclohex-1-ényl]-3,7-diméthyl-nona-2,4,6,8-tétraénoique,
- le (2E,4E,6E,8E)-9-[3-(4-Méthoxy-phénoxy)-2,6,6-triméthyl-cyclohex-1-ényl]-3,7-diméthyl-nona-2,4,6,8-tétraénal,
- l'ester tert-butylique de l'acide (2E,4E,6E,8E)-9-[3-(4-Hydroxy-phénoxy)-2,6,6-triméthyl-cyclohex-1-ényl]-3,7-diméthyl-nona-2,4,6,8-tétraénoique,
- le (2E, 4E, 6E, 8E)-9-[3-(4-hydroxy-phénoxy)-2,6,6-triméthyl-cyclohex-1-ényl]-3,7-diméthyl-nona-2,4,6,8-tétraénal,
- le (2E, 4E, 6E, 8E)-3,7-diméthyl-9-{2,6,6-triméthyl-3-diméthyl-[4-((2S, 3R, 4S, 5S, 6R)-3,4,5-trihydroxy-6-hydroxyméthyl-tétrahydro-pyran-2-yloxy)-phénoxy]-cyclohex-1-ényl}-nona-2,4,6,8-tétraénal,
- le 9-[(3-trans décènoate)-2,6,6-triméthyl-cyclohex-1-ényl]-3,7-diméthyl-nona-2,4,6,8-tétraénal,
- le 9-[(3-linoléate)-2,6,6-triméthyl-cyclohex-1-ényl]-3,7-diméthyl-nona-2,4,6,8-tétraénal,
- le 9-[(3-linolénate)-2,6,6-triméthyl-cyclohex-1-ényl]-3,7-diméthyl-nona-2,4,6,8-tétraénal,
- l'ester 3-((1E,3E,5E,7E)-3,7-diméthyl-9-oxo-nona-1,3,5,7-tétraényl)-2,4,4-triméthyl-cyclohex-2-ényl de l'acide (9Z,12Z,15Z)-Octadéca-9,12,15-triénoique,
- le 9-[(3-lipoate)-2,6,6-triméthyl-cyclohex-1-ényl]-3,7-diméthyl-nona-2,4,6,8-tétraénal,
- le 9-[{3-(8-hydroxy-5-méthyl-2-octénoate)}-2,6,6-triméthyl-cyclohex-1-ényl]-3,7-diméthyl-nona-2,4,6,8-tétraénal,
- l'ester 3-((1E,3E,5E,7E)-3,7-diméthyl-9-oxo-nona-1,3,5,7-tétraényl)-2,4,4-triméthyl-cyclohex-2-ényl de l'acide (E)-14-Hydroxy-tétradec-2-énoique,
- l'ester 3-((1E,3E,5E,7E)-3,7-diméthyl-9-oxo-nona-1,3,5,7-tétraényl)-2,4,4-triméthyl-cyclohex-2-ényl de l'acide (E)-10-Hydroxy-dec-2-énoique,
- l'ester 3-((1E,3E,5E,7E)-3,7-diméthyl-9-oxo-nona-1,3,5,7-tétraényl)-2,4,4-triméthyl-cyclohex-2-ényl de l'acide (E)-10-Acétoxy-dec-2-énoique,
- le 9-[(3-tétraacétyl glucose)-2,6,6-triméthyl-cyclohex-1-ényl]-3,7-diméthylnona-2,4,6,8-tétraénal,
- Bis ester 3-((1E,3E,5E,7E)-3,7-diméthyl-9-oxo-nona-1,3,5,7-tétraényl)-2,4,4-triméthyl-cyclohex-2-ényl et (R)-2,8-diméthyl-2-((4R, 8R)-4,8,12-triméthyl-tridécyl)-1-benzopyran-6-yl de l'acide heptanedioique
- Bis ester 3-((1E,3E,5E,7E)-3,7-diméthyl-9-oxo-nona-1,3,5,7-tétraényl)-2,4,4-triméthyl-cyclohex-2-ényl et (R)-2,8-diméthyl-2-((4R,8R)-4,8,12-triméthyl-tridécyl)-1-benzopyran-6-yl de l'acide nonanedioique
- Bis ester 3-((1E,3E,5E,7E)-3,7-diméthyl-9-oxo-nona-1,3,5,7-tétraényl)-2,4,4-triméthyl-cyclohex-2-ényl et (R)-2,8-diméthyl-2-((4R,8R)-4,8,12-triméthyl-tridécyl)-1-benzopyran-6-yl de l'acide succinique
- Bis ester 3-((1E,3E,5E,7E)-3,7-diméthyl-9-oxo-nona-1,3,5,7-tétraényl)-2,4,4-triméthyl-cyclohex-2-ényl et (R)-2,8-diméthyl-2-((4R,8R)-4,8,12-triméthyl-tridécyl)-1-benzopyran-6-yl de l'acide pentanedioique

7. Procédé de préparation des composés de formule (I) telle que définie dans l'une des revendications 1 à 6, **caractérisé en ce que** le composé de formule (II) : 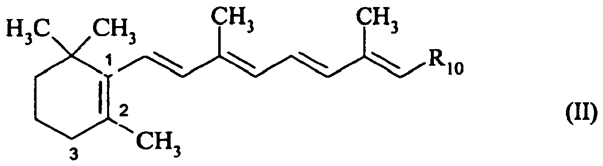 dans laquelle R₁₀ a la même signification que le radical R₁ défini dans la revendication 1, à l'exception du groupement -CH₂OH,
est soumis à une réaction d'oxydation allylique en position 3, pour conduire à un composé de formule (III) : 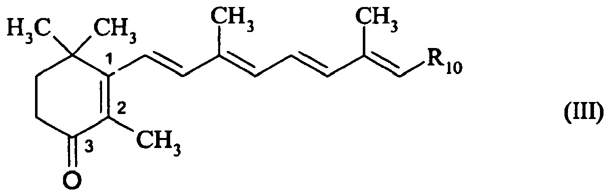 dans laquelle R₁₀ est tel que défini précédemment,
dont la fonction carbonyle en position 3 est ensuite réduite en alcool correspondant de formule (IV) : 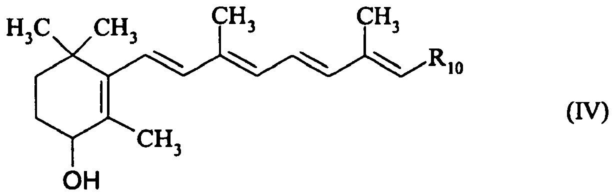 composé (IV) qui subit une réaction d'alkylation ou de couplage en milieu alcalin, acide ou neutre, éventuellement en présence d'un agent de couplage, à l'aide d'un réactif de formule R₂-X dans laquelle X représente un groupement hydroxy, ou un atome d'halogène, étant entendu que le groupement hydroxy peut être si nécessaire activé sous la forme d'un groupe partant,
pour conduire à un composé de formule (Ia) : 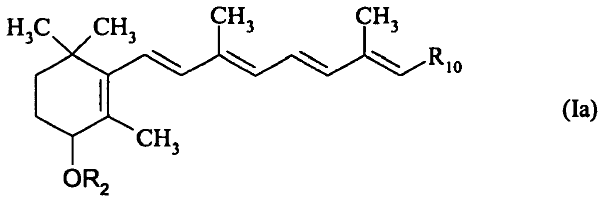 cas particulier des composés de formule (I) pour lequel R₂ est tel que défini dans la revendication 1, et R₁₀ a la même signification que précédemment,
qui, lorsque le radical R₁₀ représente un groupement -COOR₃ tel que défini dans la revendication 1, peut subir une réaction d'hydrolyse ou de réduction pour conduire au composé de formule (Ib) : 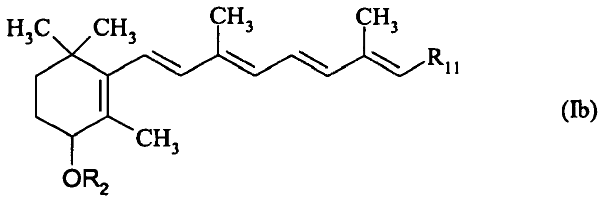 cas particulier des composés de formule (I) pour lequel R₂ est tel que défini dans la revendication 1, et R₁₁ représente un groupement -COOH, ou CH₂OH,
étant entendu que les différents groupements présents sur les composés précédents peuvent être à tout moment opportun de la synthèse protégés puis déprotégés en fonction de leurs incompatibilité avec les réactifs utilisés.

8. Utilisation d'un composé de formule (IIIa) : 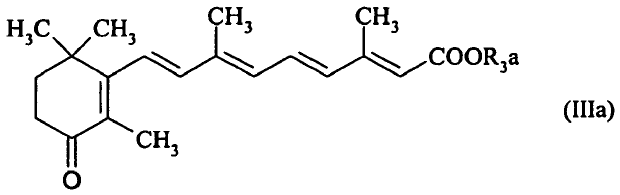 dans laquelle le groupement R₃a représente un groupement alkyle C₃-C₁₆ ramifié, comme intermédiaire de synthèse pour l'obtention de certains composés de formule (I) tels que définis dans les revendications 1 à 7.

9. Utilisation selon la revendication 8 de l'ester tert-butylique de l'acide (2E,4E,6E,8E)-9-(3-oxo-2,6,6-triméthyl-cyclohex-1-ényl)-3,7-diméthyl-nona-2,4,6,8-tétraènoique de formule IIIa.

10. Utilisation d'un composé de formule (IVa) : 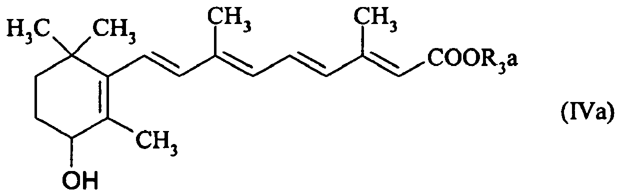 dans laquelle R₃a représente un groupement alkyle C₃-C₁₆ ramifié, comme intermédiaire de synthèse pour l'obtention de certains composés de formule (I) tels que définis dans les revendications 1 à 7.

11. Utilisation selon la revendication 10 de l'ester tert-butylique de l'acide (2E,4E,6E,8E)-9-(3-Hydroxy-2,6,6-triméthyl-cyclohex-1-ényl)-3,7-diméthyl-nona-2,4,6,8-tétraènoique de formule IVa.

12. Composition cosmétique ou dermatologique **caractérisée en ce qu'**elle comprend au moins un composé de formule (I) tel que défini dans l'une des revendications 1 à 6, dans un milieu physiologiquement acceptable.

13. Composition selon la revendication 12, **caractérisée en ce que** la quantité de composé de formule (I) varie entre 0,01 % et 5% en poids par rapport au poids total de la composition.

14. Procédé de blanchiment et/ou d'éclaircissement de la peau humaine et/ou des poils et/ou des cheveux comprenant l'application sur la peau et/ou les poils et/ou les cheveux d'une composition cosmétique contenant au moins un composé de formule (I) telle que définie selon l'une des revendications 1 à 6.

15. Utilisation d'au moins un composé de formule (I) telle que définie selon l'une des revendications 1 à 6 pour la fabrication d'une composition dermatologique destinée à dépigmenter la peau et/ou les poils et/ou les cheveux.

16. Composé de formule (I) selon les revendications 1 à 6 pour son utilisation en tant que médicament.

17. Utilisation d'au moins un composé de formule (I) selon l'une des revendications 1 à 6 pour la préparation d'une composition destinée à dépigmenter la peau.

## Claims

1. Compound of formula (I): 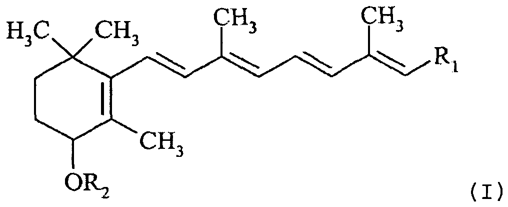 wherein
R₁ represents an R'₁ or -A-R'₁ group in which R'₁ is chosen from among -COOH, -COOR₃, -CONH₂, -CONHR₃, -CONR₃R₄, -CHO, -CH₂OH, -CH₂OR₅, and A represents a linear or branched C₁-C₁₆ alkylene group, a linear or branched C₂-C₁₆ alkenylene group or a linear or branched C₂-C₁₆ alkynylene group,
R₂ represents:
• an aryl group, optionally substituted, or heteroaryl group, optionally substituted,
• an osidic residue, or
• a fatty acid residue optionally branched and/or substituted, preferably at the end of the chain,
• an -OC-(CH₂)ₙ-CO-tocopheryl (alpha, beta, gamma or delta) group, with 2≤n≤10,
• an -R'₂-O-R₆ group, wherein R'₂ is an arylene group, optionally substituted, and R₆ represents a hydrogen atom, a linear or branched C₁-C₁₆ alkyl group, or an osidic residue,
R₃ and R₄ independently represent a linear or branched C₁-C₁₆ alkyl radical, optionally substituted, a linear or branched C₂-C₁₆ alkenyl, or a linear or branched C₂-C₁₆ alkynyl, optionally substituted,
R₅ represents a linear or branched C₁-C₁₆ alkyl radical, optionally substituted, a linear or branched C₂-C₁₆ alkenyl group, optionally substituted, a linear or branched C₂-C₁₆ alkynyl group or a linear or branched C₂-C₁₆ acyl group,
their enantiomers and diastereoisomers, as well as any salts resulting from addition to an acid or a physiologically acceptable base.

2. Compound of formula (I) according to claim 1, wherein R₁ represents a group chosen from among -COOH, -COOR₃, -CHO, -CH₂OH, -CH₂OR₅ wherein R₃ and R₅ are as defined in claim 1.

3. Compound of formula (I) according to claim 1, wherein A represents a methylene group.

4. Compound of formula (I) according to one of claims 1 to 3, wherein R₂ represents an -R'₂-O-R₆ group in which R'₂ is an arylene group, optionally substituted, and R₆ represents a hydrogen atom, a linear or branched C₁-C₆ alkyl group or an osidic residue.

5. Compound of formula (I) according to claim 4, wherein R'₂ represents a phenylene group and R₆ represents a sugar residue chosen from among glucose, galactose, fructose, mannose, fucose and rhamnose.

6. Compound of formula (I) according to claim 1 **characterized in that** it is chosen from among:
- (2E,4E,6E,8E)-3,7-dimethyl-9-{2,6,6-trimethyl-3-[4-((2S,3R,4S,5S,6R)-3,4,5-trihydroxy-6-hydroxymethyl-tetrahydro-pyran-2-yloxy)-phenoxy]-cyclohex-1-enyl}-nona-2,4,6,8-tetraenoic acid,
- tert-butyl ester of (2E,4E,6E,8E)-9-[3-(4-methoxyphenoxy)-2,6,6-trimethyl-cyclohex-1-enyl]-3,7-dimethyl-nona-2,4,6,8-tetraenoic acid,
- (2E,4E,6E,8E)-9-[3-(4-methoxy-phenoxy)-2,6,6-trimethyl-cyclohex-1-enyl]-3,7-dimethyl-nona-2,4,6,8-tetraenal,
- tert-butyl ester of (2E,4E,6E,8E)-9-[3-(4-hydroxyphenoxy)-2,6,6-trimethyl-cyclohex-1-enyl]-3,7-dimethyl-nona-2,4,6,8-tetraenoic acid,
- (2E,4E,6E,8E)-9-[3-(4-hydroxy-phenoxy)-2,6,6-trimethyl-cyclohex-1-enyl]-3,7-dimethyl-nona-2,4,6,8-tetraenal,
- (2E,4E,6E,8E)-3,7-dimethyl-9-{2,6,6-trimethyl-3-dimethyl-[4-((2S,3R,4S,5S,6R)-3,4,5-trihydroxy-6-hydroxymethyl-tetrahydro-pyran-2-yloxy)-phenoxy]-cyclohex-l-enyl}-nona-2,4,6,8-tetraenal,
- 9-[(3-trans-decenoate)-2,6,6-trimethyl-cyclohex-1-enyl]-3,7-dimethyl-nona-2,4,6,8-tetraenal,
- 9-[(3-linoleate)-2,6,6-trimethyl-cyclohex-1-enyl]-3,7-dimethyl-nona-2,4,6,8-tetraenal,
- 9-[(3-linolenate)-2,6,6-trimethyl-cyclohex-1-enyl]-3,7-dimethyl-nona-2,4,6,8-tetraenal,
- 3-((1E,3E,5E,7E)-3,7-dimethyl-9-oxo-nona-1,3,5,7-tetraenyl)-2,4,4-trimethyl-cyclohex-2-enyl ester of (9Z,12Z,15Z)-octadeca-9,12,15-trienoic acid
- 9-[(3-lipoate)-2,6,6-trimethyl-cyclohex-1-enyl]-3,7-dimethyl-nona-2,4,6,8-tetraenal,
- 9-[{3-(8-hydroxy-5-methyl-2-octenoate)}-2,6,6-trimethyl-cyclohex-1-enyl]-3,7-dimethyl-nona-2,4,6,8-tetraenal,
- 3-((1E,3E,5E,7E)-3,7-dimethyl-9-oxo-nona-1,3,5,7-tetraenyl)-2,4,4-trimethyl-cyclohex-2-enyl ester of (E)-14-hydroxy-tetradec-2-enoic acid,
- 3-((1E,3E,5E,7E)-3,7-dimethyl-9-oxo-nona-1,3,5,7-tetraenyl)-2,4,4-trimethyl-cyclohex-2-enyl ester of (E)-10-hydroxy-dec-2-enoic acid,
- 3-((1E,3E,5E,7E)-3,7-dimethyl-9-oxo-nona-1,3,5,7-tetraenyl)-2,4,4-trimethyl-cyclohex-2-enyl ester of (E)-10-acetoxy-dec-2-enoic acid,
- 9-[(3-tetraacetyl glucose)-2,6,6-trimethyl-cyclohex-1-enyl]-3,7-dimethyl-nona-2,4,6,8-tetraenal,
- 3-((1E,3E,5E,7E)-3,7-dimethyl-9-oxo-nona-1,3,5,7-tetraenyl)-2,4,4-trimethyl-cyclohex-2-enyl and (R)-2,8-dimethyl-2-((4R,8R)-4,8,12-trimethyltridecyl)-1-benzopyran-6-yl bis ester of heptanedioic acid,
- 3-((1E,3E,5E,7E)-3,7-dimethyl-9-oxo-nona-1,3,5,7-tetraenyl)-2,4,4-trimethyl-cyclohex-2-enyl and (R)-2,8-dimethyl-2-((4R,8R)-4,8,12-trimethyltridecyl)-1-benzopyran-6-yl bis ester of nonanedioic acid,
- 3-((1E,3E,5E,7E)-3,7-dimethyl-9-oxo-nona-1,3,5,7-tetraenyl)-2,4,4-trimethyl-cyclohex-2-enyl and (R)-2,8-dimethyl-2-((4R,8R)-4,8,12-trimethyl-tridecyl)-1-benzopyran-6-yl bis ester of succinic acid,
- 3-((1E,3E,5E,7E)-3,7-dimethyl-9-oxo-nona-1,3,5,7-tetraenyl)-2,4,4-trimethyl-cyclohex-2-enyl and (R)-2,8-dimethyl-2-((4R,8R)-4,8,12-trimethyl-tridecyl)-1-benzopyran-6-yl bis ester of pentanedioic acid.

7. Method for the preparation of compounds of formula (I) according to one of claims 1 to 6, **characterized in that** the compound of formula (II): 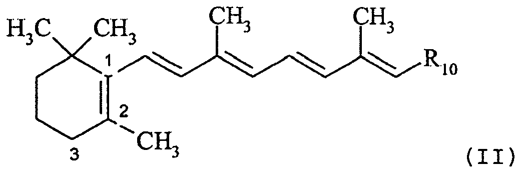 wherein R₁₀ has the same meaning as the R₁ radical according to claim 1, with the exception of the -CH₂OH group, undergoes an allylic oxidation reaction in position 3 to yield a compound of formula (III): 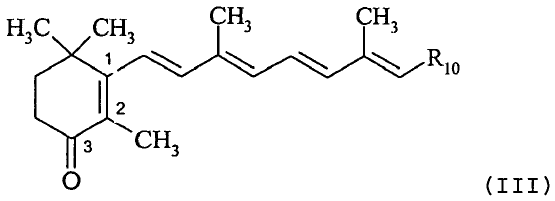 wherein R₁₀ is as defined previously, whose carbonyl group in position 3 is then reduced to the corresponding alcohol of formula (IV): 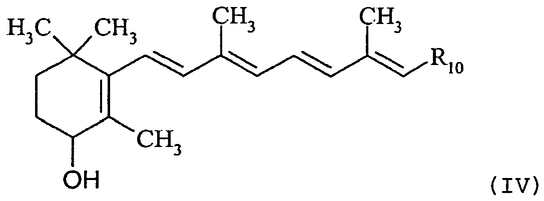 compound (IV) undergoes an alkylation reaction or coupling reaction in alkaline, acid or neutral medium, optionally in the presence of a coupling reagent, using a reagent of formula R₂-X wherein X represents a hydroxy group or a halogen atom, it being understood that the hydroxy group can be activated in the form of a parting group, if need be, to yield a compound of formula (Ia) : 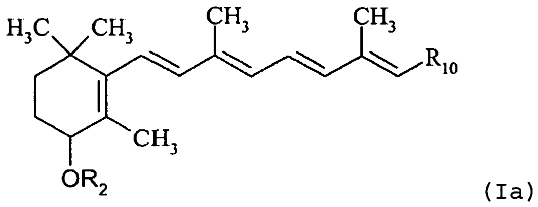 particular case of compounds of formula (I) wherein R₂ is as defined in claim 1, and R₁₀ has the same meaning as previously,
which, when the R₁₀ radical represents a -COOR₃ group as defined in claim 1, can undergo a hydrolysis or reduction reaction to yield a compound of formula (Ib): 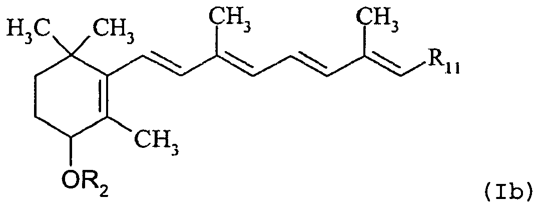 particular case of compounds of formula (I) wherein R₂ is as defined in claim 1, and R₁₁ represents a -COOH or CH₂OH group,
it being understood that the different groups present in the preceding compounds are suitable for the synthesis of protected then deprotected groups at any time, depending on their incompatibility with the reagents used.

8. Use of a compound of formula (IIIa): 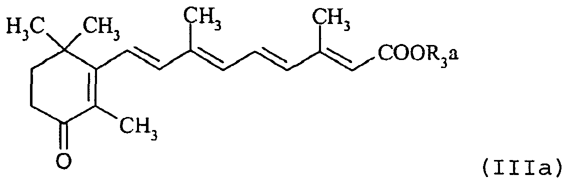 wherein the R₃a group represents a branched C₃-C₁₆ alkyl group, as a synthesis intermediate for obtaining certain compounds of formula (I) as defined in claims 1 to 7.

9. Use according to claim 8 of tert-butyl ester of (2E,4E,6E,8E)-9-(3-oxo-2,6,6-trimethyl-cyclohex-1-enyl)-3,7-dimethyl-nona-2,4,6,8-tetranoic acid of formula IIIa.

10. Use of a compound of formula (IVa): 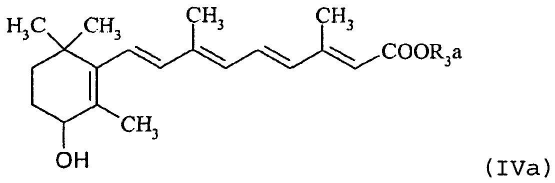 wherein the R₃a group represents a branched C₃-C₁₆ alkyl group, as a synthesis intermediate for obtaining certain compounds of formula (I) as defined in claims 1 to 7.

11. Use according to claim 10 of tert-butyl ester of (2E,4E,6E,8E)-9-(3-hydroxy-2,6,6-trimethyl-cyclohex-1-enyl)-3,7-dimethyl-nona-2,4,6,8-tetraenoic acid of formula IVa.

12. Cosmetic or dermatological composition **characterized in that** it contains at least one compound of formula (I) as defined in one of claims 1 to 6, in a physiologically acceptable medium.

13. Composition according to claim 12, **characterized in that** the amount of compound of formula (I) ranges from 0.01% to 5% by weight with respect to the total weight of the composition.

14. Method for whitening and/or lightening human skin and/or body hair and/or head hair comprising application to the skin and/or body hair and/or head hair of a cosmetic composition containing at least one compound of formula (I) as defined according to one of claims 1 to 6.

15. Use of at least one compound of formula (I) as defined according to one of claims 1 to 6, for the manufacture of a dermatological composition intended for depigmenting the skin and/or body hair and/or head hair.

16. Compound of formula (I) according to one of claims 1 to 6, for the use of same as a medication.

17. Use of at least one compound of formula (I) according to one of claims 1 to 6, for the preparation of a composition intended for depigmenting the skin.

## Patentansprüche

1. Verbindung der Formel (I): 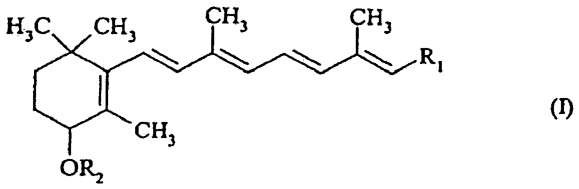 wobei:
R₁ einen Rest R'₁ oder -A-R'₁ bedeutet, wobei R'₁ aus -COOH, -COOR₃, -CONH₂, -CONHR₃, -CONR₃R₄, -CHO, -CH₂OH, -CH₂OR₅ ausgewählt ist und A einen linearen oder verzweigten C₁-C₁₆-Alkylenrest, linearen oder verzweigten C₂-C₁₆-Alkenylenrest oder linearen oder verzweigten C₂-C₁₆-Alkinylenrest bedeutet,
R₂:
• einen gegebenenfalls substituierten Arylrest oder gegebenenfalls substituierten Heteroarylrest,
• einen Saccharidrest oder
• einen gegebenenfalls verzweigten und/oder vorzugsweise am Kettenende substituierten Fettsäurerest,
• einen -OC-(CH₂)ₙ-CO-Tocopherylrest (alpha, beta oder gamma oder delta), wobei 2 ≤ n ≤ 10,
• einen Rest -R'₂-O-R₆, wobei R'₂ ein gegebenenfalls substituierter Arylenrest ist und R₆ ein Wasserstoffatom, einen linearen oder verzweigten C₁-C₆-Alkylrest oder einen Saccharidrest bedeutet,
bedeutet,
R₃ und R₄ unabhängig einen linearen oder verzweigten, gegebenenfalls substituierten C₁-C₁₆-Alkylrest, linearen oder verzweigten, gegebenenfalls substituierten C₂-C₁₆-Alkenylrest oder linearen oder verzweigten, gegebenenfalls substituierten C₂-C₁₆-Alkinylrest bedeuten,
R₅ einen linearen oder verzweigten, gegebenenfalls substituierten C₁-C₁₆-Alkylrest, linearen oder verzweigten, gegebenenfalls substituierten C₂-C₁₆-Alkenylrest, linearen oder verzweigten C₂-C₁₆-Alkinylrest oder linearen oder verzweigten C₂-C₁₆-Acylrest bedeutet,
ihre Enantiomere, Diastereoisomere sowie gegebenenfalls ihre physiologisch verträglichen Säure- oder Baseadditionssalze.

2. Verbindung der Formel (I) nach Anspruch 1, **dadurch gekennzeichnet, dass** R₁ einen aus -COOH, -COOR₃, -CHO, -CH₂OH, -CH₂OR₅ ausgewählten Rest bedeutet, wobei R₃ und R₅ wie in Anspruch 1 definiert sind.

3. Verbindung der Formel (I) nach Anspruch 1, **dadurch gekennzeichnet, dass** A einen Methylenrest bedeutet.

4. Verbindung der Formel (I) nach einem der Ansprüche 1 bis 3, **dadurch gekennzeichnet, dass** R₂ einen Rest -R'₂-O-R₆ bedeutet, wobei R'₂ ein gegebenenfalls substituierter Arylenrest ist und R₆ ein Wasserstoffatom, einen linearen oder verzweigten C₁-C₆-Alkylrest oder einen Saccharidrest bedeutet.

5. Verbindung der Formel (I) nach Anspruch 4, **dadurch gekennzeichnet, dass** R'₂ einen Phenylenrest bedeutet und R₆ einen aus Glucose, Galactose, Fructose, Mannose, Fucose und Rhamnose ausgewählten Zuckerrest bedeutet.

6. Verbindung der Formel (I) nach Anspruch 1, **dadurch gekennzeichnet, dass** sie ausgewählt ist aus:
- (2E,4E,6E,8E)-3,7-Dimethyl-9-{2,6,6,-trimethyl-3-[4-((2S,3R,4S,5S,6R)-3,4,5-trihydroxy-6-hydroxymethyl-tetrahydro-pyran-2-yloxy)-phenoxy]-cyclohex-1-enyl}-nona-2,4,6,8-tetraensäure,
- (2E,4E,6E,8E)-9-[3-(4-Methoxy-phenoxy)-2,6,6-trimethyl-cyclohex-1-enyl]-3,7-dimethyl-nona-2,4,6,8-tetraensäure-tert-butylester,
- (2E,4E,6E,8E)-9-[3-(4-Methoxy-phenoxy)-2,6,6-trimethyl-cyclohex-1-enyl]-3,7-dimethyl-nona-2,4,6,8-tetraenal,
- (2E,4E,6E,8E)-9-[3-(4-Hydroxy-phenoxy)-2,6,6-trimethyl-cyclohex-1-enyl]-3,7-dimethyl-nona-2,4,6,8-tetraensäure-tert-butylester,
- (2E,4E,6E,8E)-9-[3-(4-Hydroxy-phenoxy)-2,6,6-trimethyl-cyclohex-1-enyl]-3,7-dimethyl-nona-2,4,6,8-tetraenal,
- (2E,4E,6E,8E)-3,7-Dimethyl-9-{2,6,6-trimethyl-3-dimethyl-[4-((2S, 3R, 4S, 5S, 6R)-3,4,5-trihydroxy-6-hydroxymethyl-tetrahydro-pyran-2-yloxy)-phenoxy]-cyclohex-1-enyl}-nona-2,4,6,8-tetraenal,
- 9-[(3-trans-Decenoat)-2,6,6-trimethyl-cyclohex-1-enyl]-3,7-dimethyl-nona-2,4,6,8-tetraenal,
- 9-[(3-Linoleat)-2,6,6-trimethyl-cyclohex-1-enyl]-3,7-dimethyl-nona-2,4,6,8-tetraenal,
- 9-[(3-Linolenat)-2,6,6-trimethyl-cyclohex-1-enyl]-3,7-dimethyl-nona-2,4,6,8-tetraenal,
- 3-((1E,3E,5E,7E)-3,7-Dimethyl-9-oxo-nona-1,3,5,7-tetraenyl)-2,4,4-trimethylcyclohex-2-enylester von (9Z, 12Z, 15Z)-Octadeca-9,12,15-triensäure,
- 9-[(3-Lipoat)-2,6,6-trimethyl-cyclohex-1-enyl]-3,7-dimethyl-nona-2,4,6,8-tetraenal,
- 9-[{3-(8-Hydroxy-5-methyl-2-octenoat)}-2,6,6-trimethyl-cyclohex-1-enyl]-3,7-dimethyl-nona-2,4,6,8-tetraenal,
- 3-((1E,3E,5E,7E)-3,7-Dimethyl-9-oxo-nona-1,3,5,7-tetraenyl)-2,4,4-trimethylcyclohex-2-enylester von (E)-14-Hydroxy-tetradec-2-ensäure,
- 3-((1E,3E,5E,7E)-3,7-Dimethyl-9-oxo-nona-1,3,5,7-tetraenyl)-2,4,4-trimethylcyclohex-2-enylester von (E)-10-Hydroxy-dec-2-ensäure,
- 3-((1E,3E,5E,7E)-3,7-Dimethyl-9-oxo-nona-1,3,5,7-tetraenyl)-2,4,4-trimethylcyclohex-2-enylester von (E)-10-Acetoxy-dec-2-ensäure,
- 9-[(3-Tetraacetylglucose)-2,6,6-trimethyl-cyclohex-1-enyl]-3,7-dimethyl-nona-2,4,6,8-tetraenal,
- 3-((1E,3E,5E,7E)-3,7-Dimethyl-9-oxo-nona-1,3,5,7-tetraenyl)-2,4,4-trimethylcyclohex-2-enyl- und (R)-2,8-Dimethyl-2-((4R,8R)-4,8,12-trimethyl-tridecyl)-1benzopyran-6-yl-Bisester von Heptandisäure,
- 3-((1E,3E,5E,7E)-3,7-Dimethyl-9-oxo-nona-1,3,5,7-tetraenyl)-2,4,4-trimethylcyclohex-2-enyl- und (R)-2,8-Dimethyl-2-((4R,8R)-4,8,12-trimethyl-tridecyl)-1-benzopyran-6-yl-Bisester von Nonandisäure,
- 3-((1E,3E,5E,7E)-3,7-Dimethyl-9-oxo-nona-1,3,5,7-tetraenyl)-2,4,4-trimethylcyclohex-2-enyl- und (R)-2,8-Dimethyl-2-((4R,8R)-4,8,12-trimethyl-tridecyl)-1-benzopyran-6-yl-Bisester von Bernsteinsäure,
- 3-((1E,3E,5E,7E)-3,7-Dimethyl-9-oxo-nona-1,3,5,7-tetraenyl)-2,4,4-trimethylcyclohex-2-enyl- und (R)-2,8-Dimethyl-2-((4R,8R)-4,8,12-trimethyl-tridecyl)-1-benzopyran-6-yl-Bisester von Pentandisäure.

7. Verfahren zur Herstellung von Verbindungen der Formel (I), wie in einem der Ansprüche 1 bis 6 definiert, **dadurch gekennzeichnet, dass** die Verbindung der Formel (II): 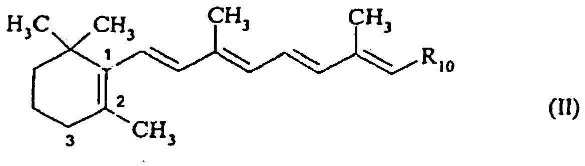 wobei R₁₀ die gleiche Bedeutung wie der in Anspruch 1 definierte Rest R₁ hat, mit Ausnahme des Rests -CH₂OH,
einer Allyloxidationsreaktion in Position 3 unterzogen wird, um eine Verbindung der Formel (III) zu ergeben: 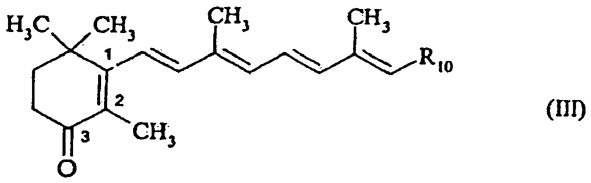 wobei R₁₀ wie vorstehend definiert ist,
deren Carbonylfunktion in Position 3 anschließend zu einem Alkohol der Formel (IV) reduziert wird: 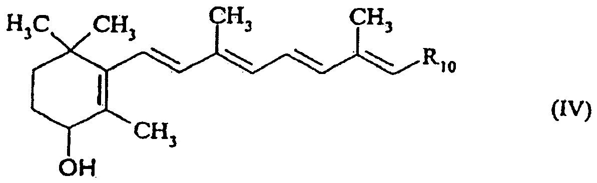 wobei Verbindung (IV) einer Alkylierungs- oder Kupplungsreaktion in alkalischem, saurem oder neutralem Milieu, gegebenenfalls in Gegenwart eines Kupplungsmittels, mit Hilfe eines Reagens der Formel R₂-X, wobei X einen Hydroxyrest oder ein Halogenatom bedeutet, unterzogen wird, mit der Maßgabe, dass der Hydroxyrest falls erforderlich in Form einer Abgangsgruppe aktiviert werden kann,
um eine Verbindung der Formel (Ia) zu ergeben: 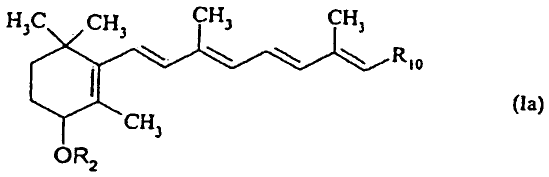 einem speziellen Fall von Verbindungen der Formel (I), bei dem R₂ wie in Anspruch 1 definiert ist und R₁₀ die gleiche Bedeutung wie vorstehend hat,
die, wenn der Rest R₁₀ einen wie in Anspruch 1 definierten Rest -COOR₃ bedeutet, einer Hydrolyse- oder Reduktionsreaktion unterzogen werden kann, um die Verbindung der Formel (Ib) zu ergeben: 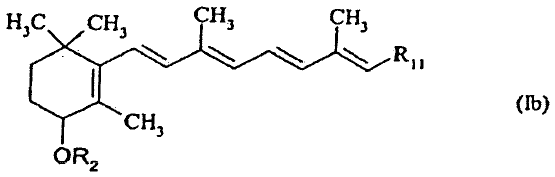 einem speziellen Fall von Verbindungen der Formel (I), bei dem R₂ wie in Anspruch 1 definiert ist und R₁₁ einen Rest -COOH oder CH₂OH bedeutet,
mit der Maßgabe, dass die an den vorstehenden Verbindungen vorhandenen unterschiedlichen Reste zu jedem geeigneten Zeitpunkt der Synthese geschützt und dann entschützt werden können, abhängig von ihrer Inkompatibilität mit den verwendeten Reagenzien.

8. Verwendung einer Verbindung der Formel (IIIa): 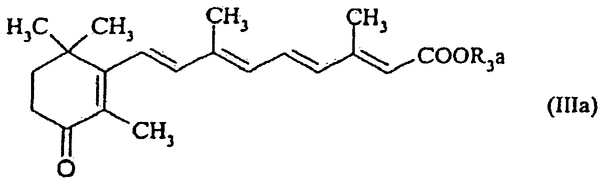 wobei der Rest R₃a einen verzweigten C₃-C₁₆-Alkylrest bedeutet, als Zwischenprodukt der Synthese, um bestimmte wie in den Ansprüchen 1 bis 7 definierte Verbindungen der Formel (I) zu erhalten.

9. Verwendung nach Anspruch 8 von (2E,4E,6E,8E)-9-(3-Oxo-2,6,6-trimethyl-cyclohex-1-enyl)-3,7-dimethyl-nona-2,4,6,8-tetraensäure-tert-butylester der Formel IIIa.

10. Verwendung einer Verbindung der Formel (IVa): 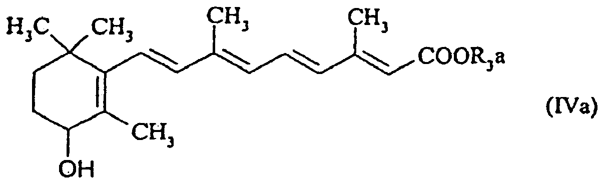 wobei R₃a einen verzweigten C₃-C₁₆-Alkylrest bedeutet, als Zwischenprodukt der Synthese, um bestimmte wie in den Ansprüchen 1 bis 7 definierte Verbindungen der Formel (I) zu erhalten.

11. Verwendung nach Anspruch 10 des (2E,4E,6E,8E)-9-(3-Hydroxy-2,6,6-trimethylcyclohex-1-enyl)-3,7-dimethyl-nona-2,4,6,8-tetraensäure-tert-butylesters der Formel IVa.

12. Kosmetische oder dermatologische Zusammensetzung, **dadurch gekennzeichnet, dass** sie mindestens eine wie in einem der Ansprüche 1 bis 6 definierte Verbindung der Formel (I) in einem physiologisch verträglichen Milieu umfasst.

13. Zusammensetzung nach Anspruch 12, **dadurch gekennzeichnet, dass** die Menge der Verbindung der Formel (I) zwischen 0,01 und 5 Gew.-%, bezogen auf das Gesamtgewicht der Zusammensetzung, variiert.

14. Verfahren zum Bleichen und/oder Aufhellen menschlicher Haut und/oder von Körperhaaren und/oder Kopfhaaren, umfassend das Aufbringen auf die Haut und/oder die Körperhaare und/oder die Kopfhaare einer kosmetischen Zusammensetzung, die mindestens eine wie in einem der Ansprüche 1 bis 6 definierte Verbindung der Formel (I) enthält.

15. Verwendung mindestens einer wie in einem der Ansprüche 1 bis 6 definierten Verbindung der Formel (I) zur Herstellung einer zur Depigmentierung der Haut und/oder der Körperhaare und/oder der Kopfhaare bestimmten dermatologischen Zusammensetzung.

16. Verbindung der Formel (I) nach den Ansprüchen 1 bis 6 zur Verwendung als Medikament.

17. Verwendung mindestens einer Verbindung der Formel (I) nach einem der Ansprüche 1 bis 6 zur Herstellung einer zur Depigmentierung von Haut bestimmten Zusammensetzung.
